# EUROPEAN PATENT APPLICATION

(11) **EP 4 799 628 A2**
(43) Date of publication of application: **02.09.2026**
(21) Application number: 26191799.1
(22) Date of filing: 01.05.2024
(51) Int. Cl.: A61K 31/7056

(54) **CHAGA MUSHROOM-RELATED FORMULATIONS, CHEMICAL COMPOUNDS, COMPOSITIONS AND METHODS OF PRODUCTION AND USE THEREOF**

(30) Priority: 02.05.2023 US 202363463443 P; 12.03.2024 US 202463564280 P
(62) Document number(s) of the earlier application(s) in accordance with Art. 76 EPC: 24800496.2 / 4 547 682
(71) Applicant: Gerosynth Laboratories, Inc., Lakeville, Minnesota 55044 (US)
(72) Inventor: O'DELL, Alisha S., Lakeville, 55044 (US)
(74) Representative: Henderson, Helen Lee

(57) **Abstract**

Medicinal and nutraceutical formulations based on Chaga mushrooms, chemical compounds and pharmaceutical compositions, and methods of production. When administered to a human or animal, the formulations, compounds, and pharmaceutical compositions are useful for the treatment of diseases such as cancers. The nutraceutical formulation includes a reduction of Chaga mushroom, where the reduction is formed in a reduction solvent; and an extraction of Chaga mushroom, where the extraction is formed in an extraction solvent. The reduction and extraction are mixed together or combined in accordance with methods known to those skilled in the art, to form the nutraceutical formulation. The medicinal formulation is a reaction product made by preparing a Chaga mushroom reduction in a reduction solvent under pressure, preparing a Chaga mushroom extraction in an extraction solvent, mixing or combining the Chaga reduction with the Chaga extraction, mixing or combining the Chaga formulation with an esterification mixture containing proline, fructose, and a fatty acid for the development of esterified compounds, and including sources of sugar esters capable of emulsion within otherwise non-miscible liquid compounds, or a predetermined amount of Manuka honey sufficient to cause esterification and facilitate emulsion at a temperature capable of inducing a reaction between the constituents of the Chaga mushroom with the constituents of the esterification mixture or Manuka honey, and combining that heterogenous mixture with medium chain triglyceride oil.

## Description

### FIELD

The present invention relates generally to nutraceutical and medicinal formulations based on Chaga mushrooms, also relates to chemical compounds and pharmaceutical compositions, and methods of production and use thereof and more specifically relates to formulations, chemical compounds, and compositions useful for the treatment of diseases such as cancers.

### BACKGROUND OF THE INVENTION

Here follows a preliminary discussion of the invention, which is not necessarily prior art, and should not necessarily be so construed.

Cancer is a leading cause of death worldwide. Additionally, an alarming rate of new cancer diagnosis in people at increasingly younger ages continues to threaten public health and quality of life. Current treatments for the human population can be effective, but there is still a significant failure rate with conventional treatments, many anti-cancer drugs currently available are not target specific and produce several side effects and complications, and the threat to long term health and quality of life remains.

Dermatologic disease and various equine cancers are growing at an unprecedented rate in various species. It is expected that there will be a 75%-90% increase in equine cancer diagnosis annually by 2030. There are no long-term treatment options for equines for advanced-stage cancer.

Therefore, there is a critical need for novel effective and less toxic therapeutic approaches to cancer treatment for both humans and animals, including equine animals.

Medicinal mushrooms and other natural products have been used to treat various diseases and infections for hundreds of years around the world. Today, medicinal mushrooms are also used to treat many types of diseases including lung diseases and many types of cancer around the world. For more than 30 years, medicinal mushrooms have been approved as an addition to standard cancer treatments in Japan and China. In these countries, mushrooms have been used safely for a long time, either alone or combined with radiation or various chemotherapeutics. More than 100 different types of mushrooms are used to treat cancer in Asia, including *Ganoderma lucidum* (relish), *Trametes versicolor* or *Curious versicolor* (turkey tail), *Lentinus edodes* (shiitake), and *Grifola frondosa* (maitake). Since many mushrooms are edible and non-toxic, they provide the possibility that one or more of their constituent compounds may provide an effective, non-toxic treatment for cancer.

Of particular interest is how mushrooms affect the immune system and if they stop or slow the growth of tumors or kill tumor cells. It is thought that certain chemical compounds, such as polysaccharides (beta-glucans), triterpenes, alkaloids and other forms of biomolecules strengthen the immune system to fight cancer directly as well as exert direct anticancer functions on the cancer cells themselves. Their potential uses, both individually and as adjuncts to cancer therapy have emerged. Mushrooms are also known to complement chemotherapy and radiation therapy by countering the side-effects of cancer, such as nausea, bone marrow suppression, anemia, and lowered resistance. Recently, a number of bio-active molecules, including anti-tumor agents, have been identified from various mushrooms. These bioactive molecules include polysaccharides, proteins, fats, ash, glycosides, alkaloids, volatile oils, tocopherols, phenolics, flavonoids, carotenoids, folates, ascorbic acid enzymes and organic acids. Polysaccharides, such as Beta-D-glucan, are the widest known mushroom-derived compounds with anti-cancer and immunomodulating properties. For example, polysaccharide K (PSK), a protein-bound polysaccharide found in turkey tail mushrooms, is an approved mushroom product used to treat cancer in Japan and has been studied as an adjuvant therapy in the treatment of gastric (stomach) cancer, breast cancer, colorectal cancer and lung cancer.

A dietary supplement prepared from *Trametes versicolor,* the turkey tail mushroom, has been shown to reduce the growth of hormone responsive prostate cancer LNCaP cells. A crude extract of *T. versicolor* has been shown to inhibit growth in a number of human cancer cell lines, including gastric cancer (7907), lung cancer (SPC), leukemia (MCL) and lymphoma (SLY). The polysaccharide of this mushroom has been shown to inhibit the proliferation of human hepatoma cancer (QCY) cells *in vitro* and *in vivo,* which occurred with apoptosis and a decrease in the expression of the cell cycle-related genes, *p53, Bcl-2* and *Fas.*

The genus *Ganoderma,* commonly known as Reishi or Lingzhi, has traditionally been administered throughout Asia as an anti-cancer agent for centuries. Extracts of *G. lucidum* have been shown to decrease the viability of human gastric carcinoma cells. Ganoderic acid T (GA-T) has been shown to inhibit tumor invasion and metastasis in human colon cancer cells lines, while other ganoderic acids, including (GA-Me, GA-Mf, GA-S) have been shown to be cytotoxic to human colon carcinoma cells and to decrease cell population growth in human carcinoma cell lines. A native glycopeptide, LZ-D-4, purified from the fruiting bodies of *G. lucidum* and its sulfated derivative, LZ-D, showed anti-tumor activity *in vitro* against mouse lymphocytic leukemia.

A d-glucan purified from *Grifola frondosa,* known as the dancing mushroom or Maitake, has been shown to enhance the efficacy of cisplatin, checking the decrease in the number of immunocompetent cells, namely macrophages, DCs and NK cells in cisplatin-treated mice. A chemically sulfated polysaccharide (S-GAP-P) derived from a water insoluble polysaccharide of

*G. frondosa*, has been shown to have anti-cancer effects when used in combination with 5- fluorouracil (5-FU) in human carcinoma cells, inhibiting cell growth and inducing cell apoptosis. A polysaccharide-peptide, GFPPS1b, isolated from cultured mycelia of G. frondose was shown to have anti-tumor activity which inhibited the proliferation of human gastric adenocarcinoma cells. The cells succumbed to apoptosis, which was associated with a drop in mitochondrial transmembrane potential, up-regulation of Bax, down regulation of Bsl-2 and activation of caspase-3.

Several different additional mushroom types have been the subject of anti-cancer studies. A water and ethanol extract of one of these, the Chaga mushroom *(Inonotus obliquus),* has been shown to induce apoptosis in human colon cancer (DLD-1) cells by prevention of reactive oxygen species (ROS)-induced tissue damage, among other functions. A water extract of Chaga was also shown to arrest the cell cycle at the Go/G1 phase in B16-F10 murine melanoma cells, causing not only apoptosis, but also induced cell differentiation. These effects were associated with the down-regulation of pRb, p53 and p27 expression levels, and further shows that the Chaga extract resulted in a G_{O}/G₁ cell cycle arrest with reduction of cyclin E/D1 and Cdk 2/4 expression levels. Furthermore, the anti-tumor effect of Chaga extract was assessed *in vivo* in Balb/c mice. Intraperitoneal administration of Chaga extract significantly inhibited the growth of tumor mass in B16-F10 cells implanted in mice, resulting in 3-fold inhibition at a dose of 20 mg/kg/day for 10 days. The ethanolic extract of sclerotium and fruiting body of Chaga elicited significant anti-tumor activity 74.6 % and 44.2% respectively.

The pentacyclic triterpenoid betulinic acid occurs naturally in Chaga and has been the subject of a number of studies for its anti-cancer properties due to its anti-tumoral activity and the ability to overcome resistance by inducing apoptosis in a variety of human cancers. Its selective cytotoxicity against cancer was first described in human melanoma both *in vitro* and *in vivo* in 1995. Since then, betulinic acid has been reported to be effective on a number of human cancers, including cancers of the lung, colon, prostate, and ovary. One study has shown that normal cells remain unaffected by betulinic acid treatment. Betulinic acid has also been applied *in vitro* in childhood cancers, *viz*. medulloblastoma, glioblastoma, Ewing sarcoma, neuroblastoma, and leukemia. Accumulated experimental evidence shows that betulinic acid treatment results in morphological change in sensitive cells, such as cell shrinkage, DNA fragmentation, nuclear condensation, and membrane blebbing. While the exact molecular mechanism underlying betulinic acid-induced apoptosis remains unclear, several studies suggest that the proteolytic cleavage of caspases, the activation of the MAP kinase cascade, the modulation of NF-KB signaling, the generation of reactive oxygen species, and the inhibition of topoisomerase I may all be contributing processes. Along with these functions, betulinic acid has also been found to reactivate the mitochondria, and induce apoptosis by releasing cytochrome C, a signaling protein that induces apoptosis naturally in response to irreparable cellular damage.

There is a need to further explore the potential benefits of Chaga mushrooms, particularly the potential oncological benefits. One limitation discovered in the continuing research of betulinic acid, and discussed openly among many research groups, is the poor bioavailability of betulinic acid. Scientists have speculated that this low bioavailability is caused by the poor water solubility of betulinic acid. The low bioavailability of betulinic severely limits practical applications of betulinic acid as a therapeutic agent. Various attempts to overcome this low bioavailability have been tried and have included self-nanoemulsifying drug delivery systems and spray gun technologies, but none have provided a practical solution. Therefore, what is needed is a betulinic acid or derivative thereof having sufficient bioavailability to provide a therapeutic effect when administered to humans and animals.

### SUMMARY

Nutraceutical and medicinal formulations, chemical compounds and pharmaceutical compositions are provided herein. Also provided are methods of making the formulations and methods of using the formulations, chemical compounds, and pharmaceutical compositions to treat diseases such as cancer.

One embodiment of the invention is directed to a nutraceutical formulation and method of producing the nutraceutical formulation, wherein the formulation is a reduction of Chaga mushroom, formed in a reduction solvent; and an extraction of Chaga mushroom, formed in an extraction solvent, and the reduction and extraction are mixed together, or combined in accordance with methods known to those skilled in the art, to form the nutraceutical formulation.

Another embodiment of the invention is a medicinal formulation and a method of preparing the medicinal formulation by preparing a Chaga mushroom reduction in a reduction solvent under pressure, preparing a Chaga mushroom extraction in an extraction solvent, combining the Chaga reduction with the Chaga extraction, r combining the Chaga formulation with an esterification mixture containing proline, fructose, and a fatty acid such as a medium chain triglyceride (MCT) oil or sunflower lecithin, or other carbon chain fatty acids of various carbon chain length, with and without phospholipids for the development of esterified compounds, also including sources of sugar esters capable of emulsion within otherwise non-miscible liquid compounds, or a predetermined amount of Manuka honey sufficient to cause esterification and facilitate emulsion at a temperature capable of inducing a reaction between the constituents of the Chaga mushroom with the constituents of the esterification mixture or Manuka honey, and combining that heterogenous mixture with a medium chain triglyceride to produce the medicinal formulation..

Another embodiment is a chemical compound. The chemical compound is isolated from the medicinal formulation or synthesized and is optionally combined with a pharmaceutically-acceptable carrier to form a pharmaceutical composition.

Another embodiment is a method of treating cancer by administering one or more of the medicinal formulations, chemical compounds, or pharmaceutical compositions to a human or animal.

The following are additional various concepts numbered for reference in other concepts.

Concept 1 - A Chaga formulation containing: a reduction of Chaga mushroom, where the reduction is formed in a reduction solvent; and an extraction of Chaga mushroom, where the extraction is formed in an extraction solvent; wherein the reduction and extraction are mixed together.

Concept 2 - A formulation as recited in concept 1, further containing a distillation remaining fraction formed from a mixture of the reduction and the extraction in a volume range of 1:1 to 1:10 extraction to reduction.

Concept 3 - The formulation of any of concepts 1 or 2, wherein a volume ratio of reduction to extraction (the reduction: extraction volume ratio) is in a range of 100:1 to 1: 100.

Concept 4 - The formulation of any of concepts 1-3, wherein the reduction: extraction volume ratio is in a range of 20:1 to 1:10.

Concept 5 - The formulation of any of concepts 1-4, wherein the reduction: extraction volume ratio is in a range of 20:1 to 1:1.

Concept 6 - The formulation of concept 2, wherein a volume ratio of the reduction to remaining fraction (reduction: remaining fraction volume ratio) is in a range of 100:1 to 1: 100.

Concept 7 - The formulation of concept 2 or 6, wherein the reduction: remaining fraction volume ratio is in a range of 20:1 to 1:10.

Concept 8 - The formulation of any of concepts 2, 6 or 7, wherein the reduction: remaining fraction volume ratio is in a range of20:1 to 1:1.

Concept 9 - The formulation of concept 2, wherein a volume ratio of the extraction to the remaining fraction (extraction: remaining fraction volume ratio) is in a range of 100:1 to 1:100.

Concept 10 - The formulation of concepts 2 or 9, wherein the extraction: remaining fraction volume ratio in a range of 10:1 to 1:10.

Concept 11 - The formulation of any of concepts 2, 9 or 10, wherein the extraction: remaining fraction volume ratio is in a range of 5:1 and 1:5.

Concept 12 - The formulation of concept 2, wherein the reduction, extraction and distillation remaining fraction are present in the formulation in a volume ratio of about 5:1:1.

Concept 12 - The formulation of any of concepts 1-12, wherein the reduction solvent has a dielectric constant of greater than 50.

Concept 14 - The formulation of any of concepts 1-13, wherein the reduction solvent has a dielectric constant of greater than 60.

Concept 15 - The formulation of any of concepts 1-14, wherein the reduction solvent has a dielectric constant of greater than 70.

Concept 16 - The formulation of any of concepts 1-15, wherein the extraction solvent has a dielectric constant of less than 50.

Concept 17 - The formulation of any of concepts 1-16, wherein the extraction solvent has a dielectric constant of less than 40.

Concept 18 - The formulation of any of concepts 1-17, wherein the extraction solvent has a dielectric constant of less than 30.

Concept 19 - The formulation of any of concepts 1-18, wherein the extraction solvent has a first dielectric constant and the reduction solvent has a second dielectric constant, the second dielectric constant being greater than the first dielectric constant by at least 10.

Concept 20 - The formulation of any of concepts 1-19, wherein the extraction solvent has a first dielectric constant and the reduction solvent has a second dielectric constant, the second dielectric constant being greater than the first dielectric constant by at least 20.

Concept 21 - The formulation of any of concepts 1-20, wherein the extraction solvent has a first dielectric constant and the reduction solvent has a second dielectric constant, the second dielectric constant being greater than the first dielectric constant by at least 30.

Concept 22 - The formulation of any of concepts 1-21, further containing a honey component.

Concept 23 - The formulation of concept 22, wherein the honey component contains manuka honey.

Concept 24 - The formulation of any of concepts 22 and 23, wherein the honey component is present in the formulation with a weight ratio of reduction: honey in the range 10:0.1 to 10:10.

Concept 25 - The formulation of any of concepts 22-24, wherein the honey component is present in the formulation with a weight ratio of reduction: honey in the range 10:0.1 to 10:2.

Concept 26 - The formulation of any of concepts 22-25, wherein the honey component is present in the formulation with a weight ratio of reduction: honey about 10:1.

Concept 27 - The formulation of any of concepts 1-26, further containing a carrier.

Concept 28 - The formulation of concept 26, wherein the carrier contains a medium chain triglyceride.

Concept 29 - The formulation of concept 26, wherein the carrier contains coconut oil.

Concept 30 - The formulation of any of concepts 27-29, wherein the carrier is present in the formulation at a weight ratio of reduction: carrier in the range 10:0.1 to 1:50.

Concept 31 - The formulation of any of concepts 27-30, wherein the carrier is present in the formulation at a weight ratio of reduction: carrier in the range 10:0.1 to 10:2.

Concept 32 - The formulation of any of concepts 27-31, wherein the carrier is present in the formulation at a weight ratio of reduction: carrier around 10:1.

Concept 33 - A method of preparing a formulation by preparing a Chaga reduction in a reduction solvent; preparing a Chaga extraction in an extraction solvent; and mixing the Chaga reduction with the Chaga extraction to produce the formulation.

Concept 34 - The method as recited in concept 33, further containing preparing a distillation remaining fraction from a mixture of the Chaga reduction and the Chaga extraction, the Chaga reduction and Chaga extraction being present in the mixture in a volume range of 1:1 to 1:10 extraction: reduction and mixing the Chaga reduction and Chaga extraction by mixing the Chaga reduction, Chaga extraction and distillation remaining fraction.

Concept 35 - The method of any of concepts 33-34, wherein mixing the Chaga reduction and the Chaga extraction includes mixing between 100 parts per volume reduction to 1 part per volume extraction and 1 part by volume reduction to 100 parts extract.

Concept 36 - The method of any of concepts 33-35, wherein mixing the Chaga reduction and the Chaga extraction contains mixing between 20 parts per volume reduction to 1 part per volume extraction and 1 part by volume reduction to 10 parts extract.

Concept 37 - The method of any of concepts 33-36, wherein mixing the Chaga reduction and the Chaga extraction contains mixing between 20 parts per volume reduction to 1 part per volume extraction and 1 part by volume reduction to 1 part extract.

Concept 38 - The method of concept 34, wherein mixing the Chaga reduction and the remaining distillation fraction contains mixing between 100 parts per volume reduction and 1 part per volume remaining distillation fraction and 1 part per volume reduction and 100 parts per volume remaining distillation fraction.

Concept 39 - The method of concept 34 or concept 38, wherein mixing the Chaga reduction and the remaining distillation fraction contains mixing between 20 parts per volume reduction and 1 part per volume remaining distillation fraction and 1 part per volume reduction and 10 parts per volume remaining distillation fraction.

Concept 40 - The method of any of concepts 34, 38 or 39, wherein mixing the Chaga reduction and the remaining distillation fraction contains mixing between 20 parts per volume reduction and 1 part per volume remaining distillation fraction and I part per volume reduction and 1 part per volume remaining distillation fraction.

Concept 41 - The method of any of concepts 34 or 38-40, wherein mixing the Chaga extraction and the remaining distillation fraction contains mixing between 100 parts per volume extraction and 1 part per volume remaining distillation fraction and 1 part per volume extraction and 100 parts per volume remaining distillation fraction.

Concept 42 - The method of any of concepts 34 or 38-41, wherein mixing the Chaga extraction and the remaining distillation fraction contains mixing between 10 parts per volume extraction and 1 part per volume remaining distillation fraction and 1 part per volume extraction and 10 parts per volume remaining distillation fraction.

Concept 43 - The method of any of concepts 34 or 38-42, wherein mixing the Chaga extraction and the remaining distillation fraction contains mixing between 5 parts per volume extraction and 1 part per volume remaining distillation fraction and I part per volume extraction and 50 parts per volume remaining distillation fraction.

Concept 44 - The method of concept 34, wherein the reduction, extraction and distillation remaining fraction are present in the formulation in a volume ratio of about 5:1: 1.

Concept 45 - The method of any of concepts 33-44, wherein the reduction solvent has a dielectric constant of greater than 50.

Concept 46 - The method of any of concepts 33-45, wherein the reduction solvent has a dielectric constant of greater than 60.

Concept 47 - The method of any of concepts 33-46, wherein the reduction solvent has a dielectric constant of greater than 70.

Concept 48 - The method of any of concepts 33-47, wherein the extraction solvent has a dielectric constant of less than 50.

Concept 49 - The method of any of concepts 33-48, wherein the extraction solvent has a dielectric constant of less than 40.

Concept 50 - The method of any of concepts 33-49, wherein the extraction solvent has a dielectric constant of less than 30.

Concept 51 - The method of any of concepts 33-50, wherein the extraction solvent has a first dielectric constant and the reduction solvent has a second dielectric constant, the second dielectric constant being great than the first dielectric constant by at least 10.

Concept 52 - The method of any of concepts 33-51, wherein the extraction solvent has a first dielectric constant and the reduction solvent has a second dielectric constant, the second dielectric constant being great than the first dielectric constant by at least 20.

Concept 53 - The method of any of concepts 33-52, wherein the extraction solvent has a first dielectric constant, and the reduction solvent has a second dielectric constant, the second dielectric constant being great than the first dielectric constant by at least 30.

Concept 54 - The method of any of concepts 33-53, further containing providing a honey component and wherein mixing the Chaga reduction with the Chaga extraction further contains missing the honey component with the Chaga reduction and the Chaga extraction.

Concept 55 - The method of any of concepts 33-54, wherein the honey component contains manuka honey.

Concept 56 - The method of any of concepts 33-55, wherein the honey component is present in the formulation with a weight ratio of reduction: honey in the range 10:0.1 to 10: 10.

Concept 57 - The method of any of concepts 33-56, wherein the honey component is present in the formulation with a weight ratio of reduction: honey in the range 10:0.1 to 10:2.

Concept 58 - The method of any of concepts 33-57, wherein the honey component is present in the formulation with a weight ratio of reduction: honey about 10:1.

Concept 59 - The method of any of concepts 33-58, further containing providing a carrier and wherein mixing the Chaga reduction with the Chaga extraction further contains mixing the carrier with the Chaga reduction and the Chaga extraction.

Concept 60 - The method of any of concept 59, wherein the carrier contains a medium chain triglyceride.

Concept 61 - The method of either concept 59 or 60, wherein the carrier contains coconut oil.

Concept 62 - The method of any of concepts 59-61, wherein the carrier is present in the formulation at a weight ratio of reduction: carrier in the range 10:0.1 to I :50.

Concept 63 - The method of any of concepts 59-62, wherein the carrier is present in the formulation at a weight ratio of reduction: carrier in the range 10:0.1 to 10:2.

Concept 64 - The method of any of concepts 59-63, wherein the carrier is present in the formulation at a weight ratio of reduction: carrier around 10: 1.

Concept 65 - A method of treating a mammal, containing:
administering the formulation of any of concepts 1-32 to a mammalian patient with a dose in the range of between 0.1 ounces per 1000 pounds of patient weight to I ounce per pounds of patient weight per day.

Concept 66 - The method as recited in concept 65, wherein the formulation is administered to the patient once per day.

Concept 67 - The method as recited in concept 65, wherein the formulation is administered to the patient at least twice per day.

Concept 68 - The method as recited in any of concepts 65-67, wherein the mammalian patient is a human patient.

Concept 69 - The method as recited in any of concepts 65-67, wherein the mammalian patient is an equine patient.

Concept 70 - The method as recited in any of concepts 65-69, wherein administering the formulation contains administering the formulation orally.

Concept 71 - The method as recited in any of concepts 65-69, wherein administering the formulation contains administering the formulation topically.

Concept 72 - The method as recited in any of concepts 65-69, wherein administering the formulation contains administering the formulation orally or administering the formulation topically.

Concept 73 - A chemical compound which, when administered to mammals, the compound is useful for the treatment of diseases such as cancers. The chemical compound can be isolated from the medicinal formulation or synthesized.

Concept 74 - A pharmaceutical composition containing a chemical compound in a pharmaceutically-acceptable carrier. When administered to mammals, the composition is useful for the treatment of diseases such as cancers.

### BRIEF DESCRIPTION OF THE DRAWINGS

The invention may be more completely understood in consideration of the following detailed description of various embodiments of the invention in connection with the accompanying drawings, in which:

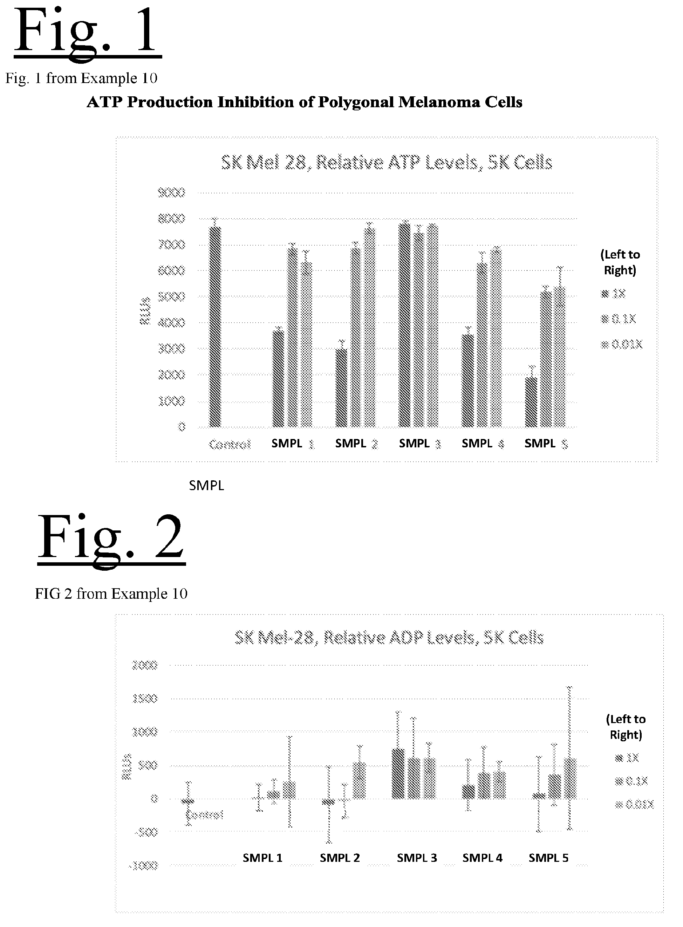

**Figures 1-7** show the results of the *in vitro* testing to understand the cytotoxic effects of the formulation on three types of cancer cell lines. The *in vitro* tests were performed on ATCC cell bank-derived cancer cell lines. The cell lines tested were polygonal melanoma, triple negative breast cancer and liver cancer cells. The cytotoxic effects, apoptosis inducing effects, and the inhibition of ATP utilization by the cancer cells were all demonstrated consistently with several *in vitro* study outcomes.
**Figure 1** is a bar graph showing ATP production inhibition of the novel products on polygonal melanoma.
**Figure 2** is a bar graph showing relative ADP levels from the ATP inhibition; this is an indicator of a type of cell death.
**Figure 3** is a bar graph showing the cytotoxic nature of the novel products on polygonal melanoma.
**Figure 4** is a bar graph showing ATP production inhibition of the novel products on triple negative breast cancer.
**Figure 5** is a bar graph showing relative ADP levels from the ATP inhibition; this is an indicator of a type of cell death.
**Figure 6** is a bar graph showing cytotoxic nature of the novel products on triple negative breast cancer.
**Figure 7** is a bar graph showing ATP production inhibition of the novel products in liver cancer
**Figure 8** is a bar graph showing relative ADP levels from the ATP inhibition; this is an indicator of type of cell death.
**Figure 9** is a bar graph showing the cytotoxic nature of the novel products on liver cancer.
**Figure 10** is a bar graph showing quantitative ATP levels of HepG2 cells following treatment with samples.
**Figure 11** is a graph providing the data of Figure 9 log transformed to better visualize the data.
**Figure 12** is a bar graph showing a HepG2 Cell Viability assessment.
**Figure 13** is a graph providing the data of Figure 12 log transformed to better visualize the data.
**Figure 14** is an IC₅₀ curve examining cell viability in HepG2 cells treated with sample 5.
**Figure 15** is an IC₅₀ curve examining cell viability in HepG2 cells treated with sample 6
**Figure 16** is a bar graph showing relative ATP levels of MDA-MB-231 cells following treatment with samples 5 or 6.
**Figure 17** is a graph providing the data of Figure 16 log transformed to better visualize the data.
**Figure 18** is a bar graph showing a Cell viability assessment of MDA-MB-231 cells using the CellQuanti-Blue^{™} Cell Viability Assay.
**Figure 19** is a graph providing the data of Figure 18 log transformed to better visualize the data.
**Figure 20** is a graph providing an IC₅₀ assessment of Sample 5 with MDA-MB-231 cells.
**Figure 21** is a graph providing an IC₅₀ assessment of Sample 6 with MDA-MB-231 cells.
**Figure 22** is a bar graph showing quantitative ATP levels of SK-Mel 28 cells folling treatment with Samples 5 and 6.
**Figure 23** is a graph providing the data of Figure 22 log transformed to better visualize the data.
**Figure 24** is a bar graph showing a cell viability assessment of SK-Mel 28 cells using the CellQuanti-Blue Cell Viability Assay.
**Figure 25** is a graph providing the data of Figure 24 log transformed to better visualize the data.
**Figure 26** is a graph providing an IC₅₀ assessment of Sample 5 with SK-Mel 28 cells.
**Figure 27** is a graph providing an IC₅₀ assessment of Sample 6 with SK-Mel 28 cells.
**Figure 28** is a graph showing quantitative ATP levels of HCT-116 cells following treatment with Sample 7.
**Figure 29** is a graph showing quantitative ATP levels of HCT-116 cells following treatment with Sample 8.
**Figure 30** is a graph providing an IC₅₀ assessment of Sample 7 with HCT-116 cells.
**Figure 31** is a graph providing an IC₅₀ assessment of Sample 8 with HCT-116 cells.
**Figure 32** is a graph showing quantitative ATP levels of HEPG2 cells following treatment with Sample 7.
**Figure 33** is a graph showing quantitative ATP levels of HEPG2 cells following treatment with Sample 8.
**Figure 34** is a graph providing an IC₅₀ assessment of Sample 7 with HEPG2 cells.
**Figure 35** is a graph providing an IC₅₀ assessment of Sample 8 with HEPG2 cells.
**Figure 36** is a graph showing quantitative ATP levels of MDA-MB-231 cells following treatment with Sample 7.
**Figure 37** is a graph showing quantitative ATP levels of MDA-MB-231 cells following treatment with Sample 8.
**Figure 38** is a graph providing an IC₅₀ assessment of Sample 7 with MDA-MB-231 cells.
**Figure 39** is a graph providing an IC₅₀ assessment of Sample 8 with MDA-MB-231 cells.
**Figure 40** is a graph showing quantitative ATP levels of SK-Mel 28 cells following treatment with Sample 7.
**Figure 41** is a graph showing quantitative ATP levels of SK-Mel 28 cells following treatment with Sample 8.
**Figure 42** is a graph providing an IC₅₀ assessment of Sample 7 with SK-Mel 28 cells.
**Figure 43** is a graph providing an IC₅₀ assessment of Sample 8 with SK-Mel 28 cells.

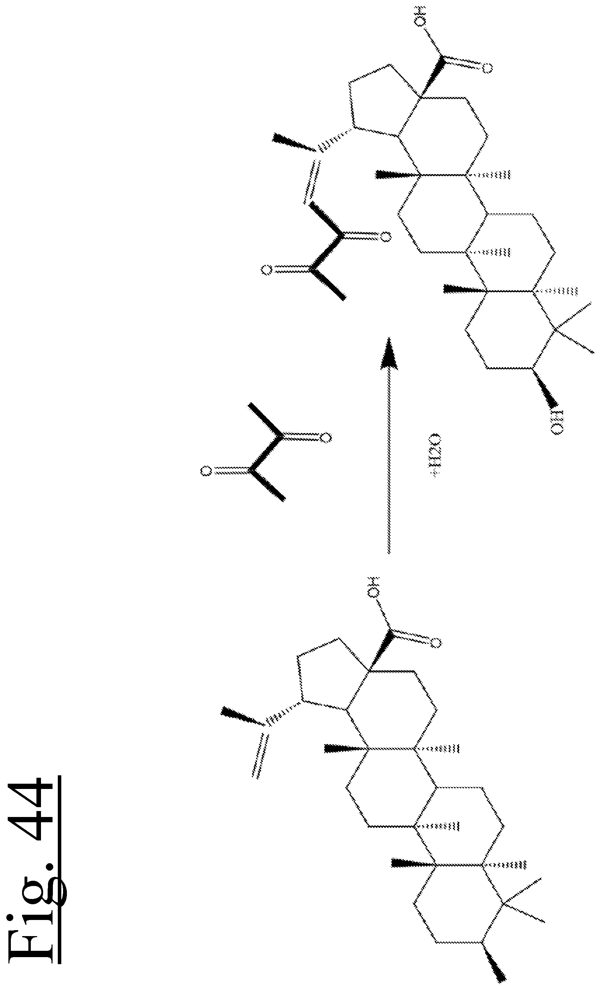

**Figure 44** provides an enlarged view of the chemical reaction synthesis pathway shown in Figure 58 for four different possible reaction products as a result of a first proposed theoretical reaction between betulinic acid (BA) and methylglyoxal (MGO) to produce compound 1.

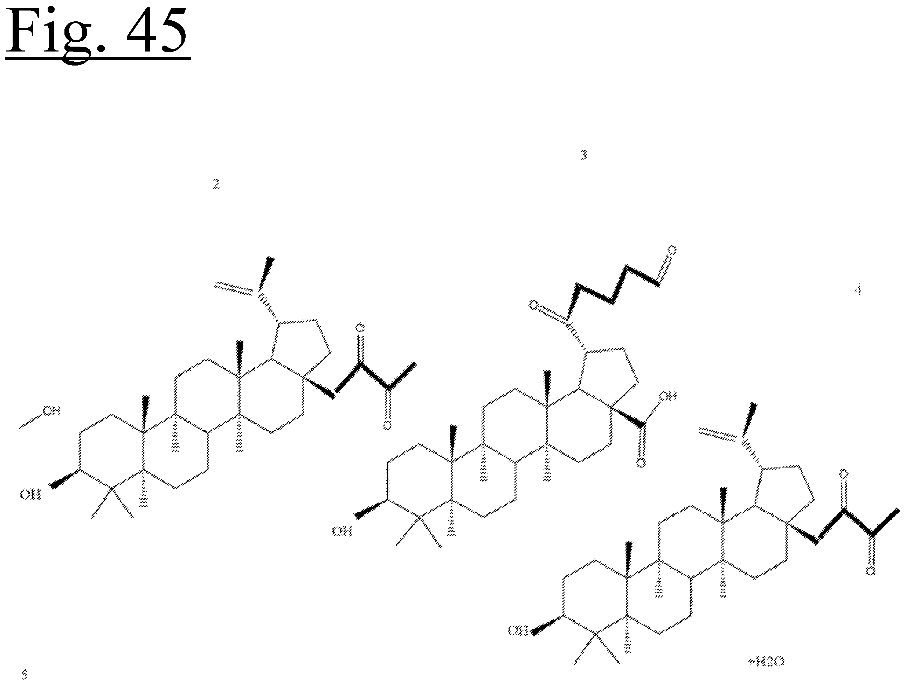

**Figure 45** provides an enlarged view of the chemical reaction synthesis pathway shown in Figure 58 for four different possible products as a result of a first proposed theoretical reaction between betulinic acid and methylglyoxal showing the three additional intermediate compounds, compounds 2, 3 and 4.

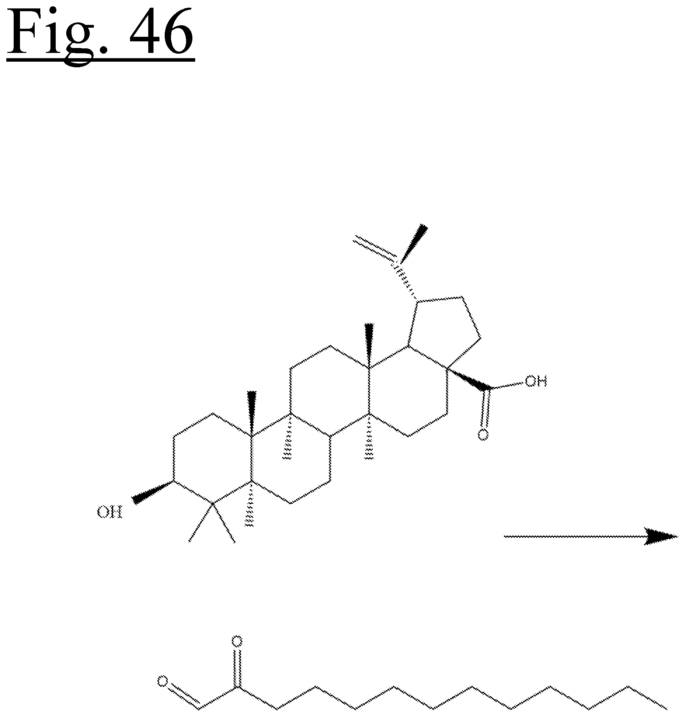

**Figure 46** provides an enlarged view of the chemical reaction synthesis pathway shown in Figure 58 for four different possible products as a result of a proposed theoretical reaction between betulinic acid (BA), methylglyoxal and the fatty acids present in MCT oil.

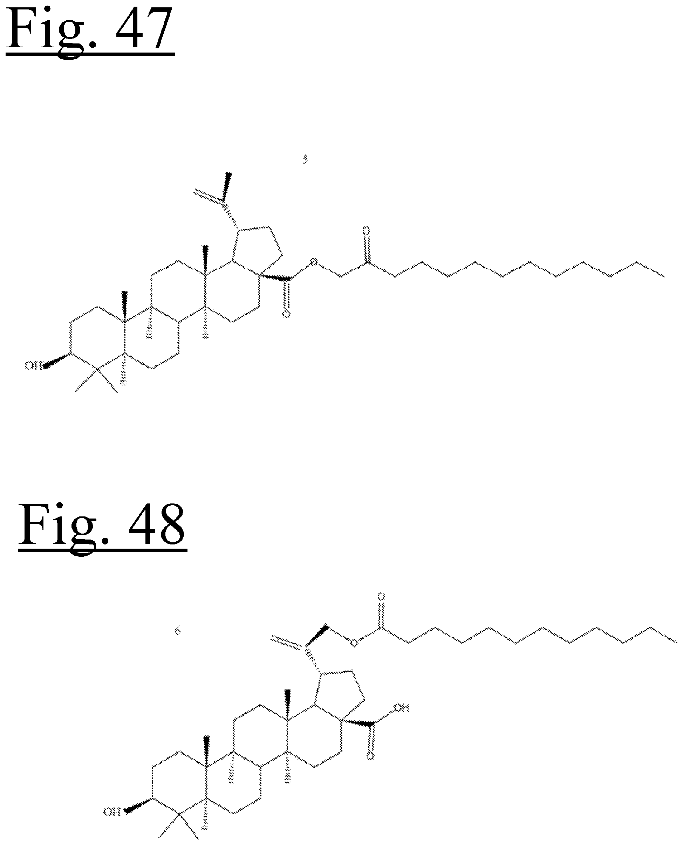

**Figure 47** provides an enlarged view of one of the four different possible products of Figure 46, compound 5.
**Figure 48** provides an enlarged view of one of the four different possible products of Figure 46, compound 6.

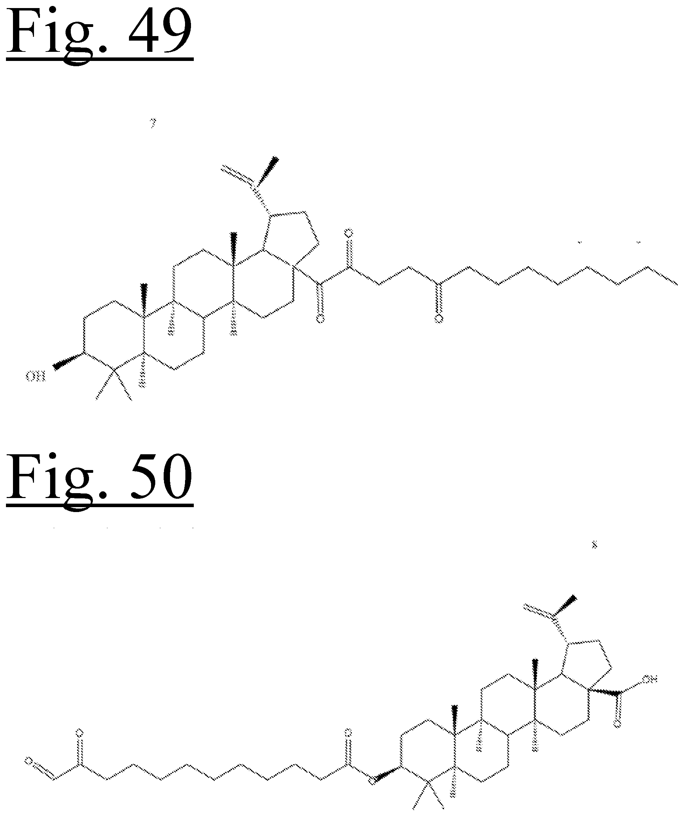

**Figure 49** provides an enlarged view of one of the four different possible products of Figure 46, compound 7.
**Figure 50** provides an enlarged view of one of the four different possible products of Figure 46, compound 8.

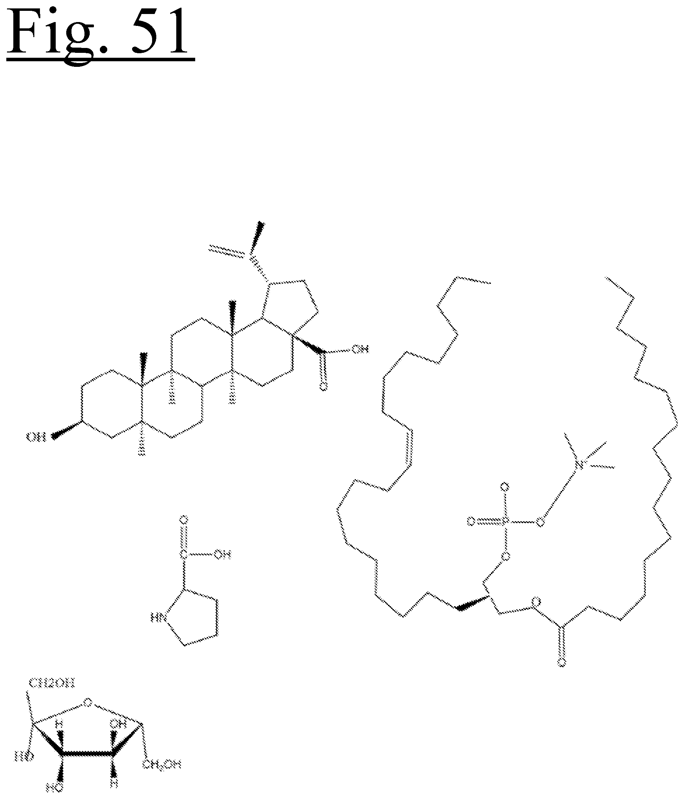

**Figure 51** provides an enlarged view of additional components of the chemical reaction synthesis pathway shown in Figure 58.

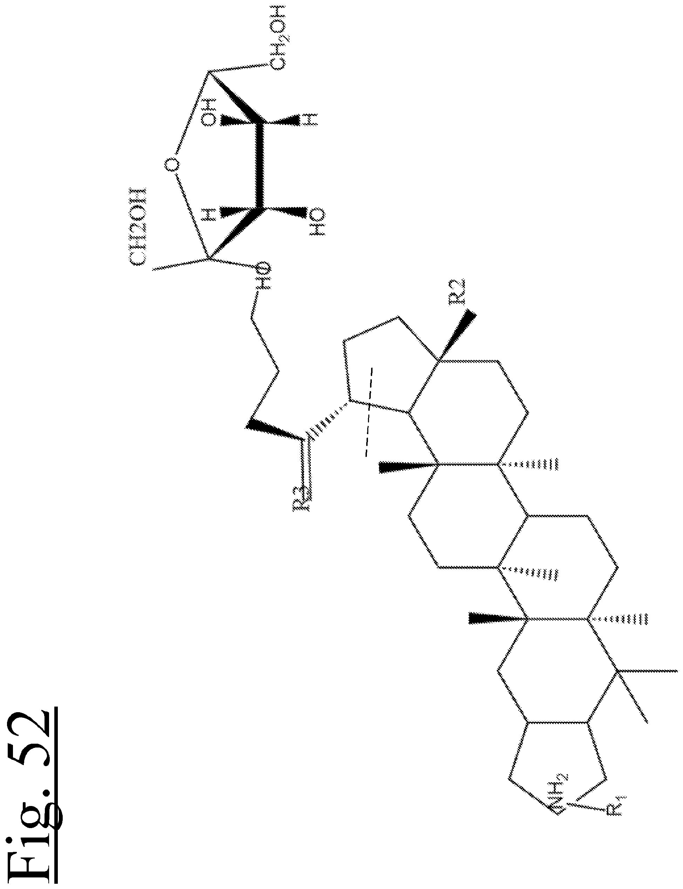

**Figure 52** provides an enlarged view of a possible product of the chemical reaction synthesis pathway shown in Figure 58.

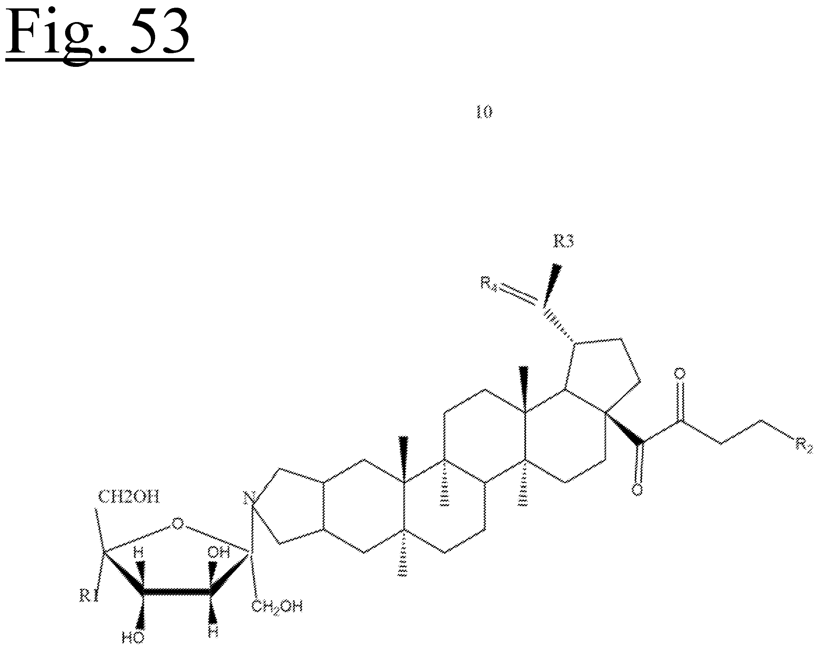

**Figure 53** provides an enlarged view of yet another possible product of the chemical reaction synthesis pathway shown in Figure 58, compound 10.

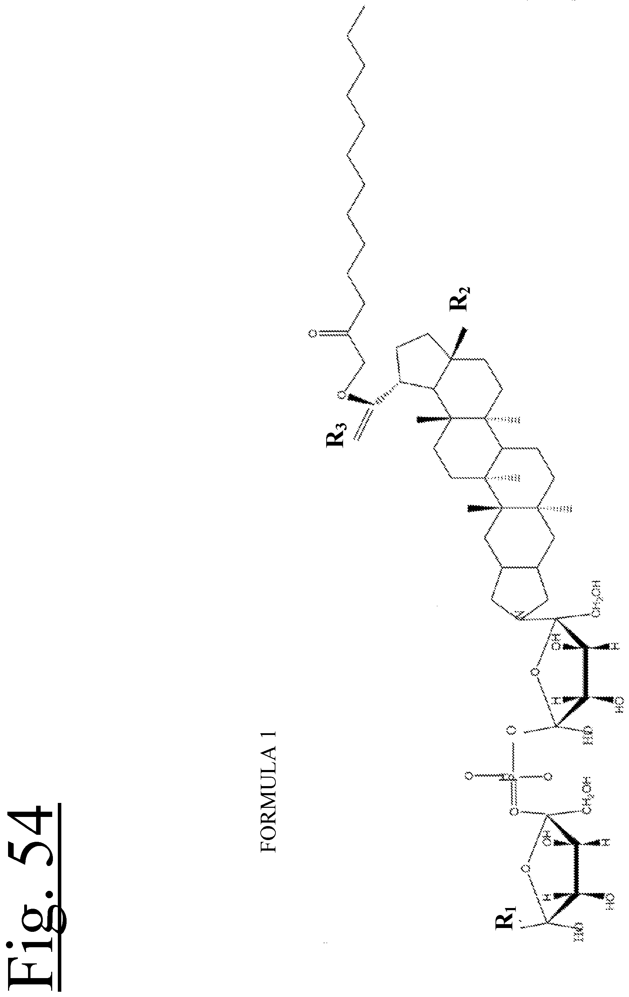

**Figure 54** provides an enlarged view of a main product of the chemical reaction synthesis pathway shown in Figure 58 as a result of a proposed theoretical reaction between betulinic acid (BA), methylglyoxal and the fatty acids present in MCT oil, compound 11 (referred to herein also as the compound represented by Formula 1).

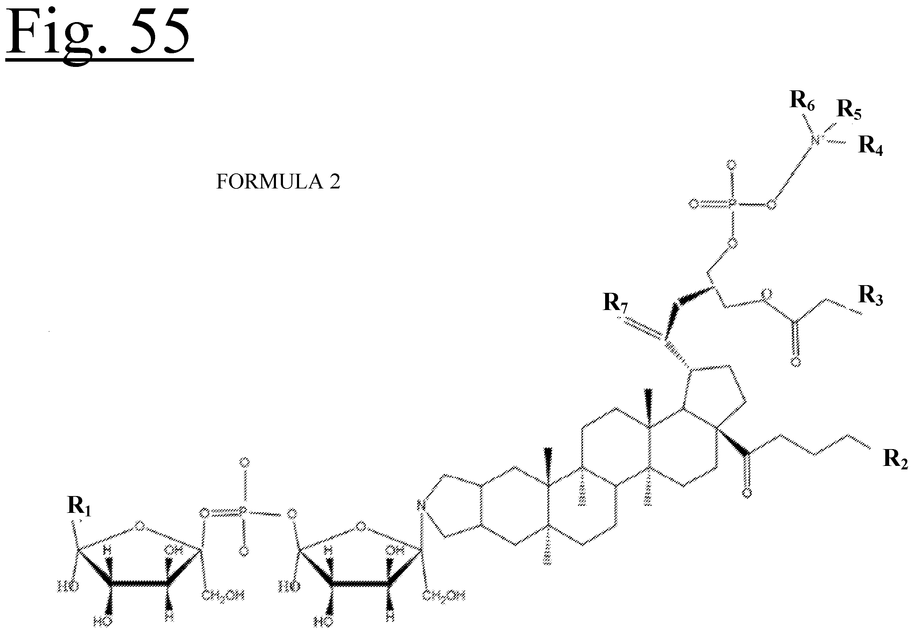

**Figure 55** provides an enlarged view of a main product of the chemical reaction synthesis pathway shown in Figure 58, as a result of a proposed theoretical reaction between betulinic acid (BA), methylglyoxal and the fatty acids present in MCT oil, compound 12. (referred to herein also as the compound represented by Formula 2).
**Figure 56** provides NMR data measurements showing unique electromagnetic signal with a frequency that is uniquely characteristic of the magnetic field of a specific nuclei; showing the presence of the specific characteristics of the compound of Formula 1.
**Figure 57** provides NMR data measurements showing unique electromagnetic signal with a frequency that is uniquely characteristic of the magnetic field of a specific nuclei; showing the presence of the specific characteristics of the compound of Formula 2.
**Figure 58** shows the chemical reaction synthesis pathway for four different products all as a result of the proposed theoretical reaction. Compounds 1-4 show the possible reaction and product outcomes of betulinic acid (BA) and methylglyoxal (MGO) in an isolated environment. Compounds 5-8 show the potential product outcomes from the proposed reaction between BA, MGO and the fatty acids present in MCT oil described above. Compounds 9-12 show the potential product outcomes from the proposed reaction between betulinic acid, proline, lecithin and fructose.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the invention as defined by the appended claims.

### DETAILED DESCRIPTION

Described herein are nutraceutical and medicinal formulations, chemical compounds and pharmaceutical compositions containing the chemical compounds. Also described are methods of making the formulations, chemical compounds and pharmaceutical compositions containing the chemical compounds, and methods of using the formulations to treat diseases such as cancers.

One embodiment of the invention is directed to a nutraceutical formulation, wherein the formulation is a reduction of Chaga mushroom, where the reduction is formed in a reduction solvent; and an extraction of Chaga mushroom, where the extraction is formed in an extraction solvent, wherein the reduction and extraction are mixed together, or combined in accordance with methods known to those skilled in the art, to form the nutraceutical formulation.

Another embodiment of the invention is a medicinal formulation and a method of preparing the medicinal formulation by preparing a Chaga mushroom reduction in a reduction solvent under pressure, preparing a Chaga mushroom extraction in an extraction solvent, mixing or combining the Chaga reduction with the Chaga extraction, mixing or combining the Chaga formulation with an esterification mixture containing proline, fructose, and a fatty acid such as medium chain triglyceride (MCT) oil or sunflower lecithin, or other carbon chain fatty acids of various carbon chain length, with and without phospholipids for the development of esterified compounds, also including sources of sugar esters capable of emulsion within otherwise non-miscible liquid compounds, or a predetermined amount of Manuka honey sufficient to cause esterification and facilitate emulsion at a temperature capable of inducing a reaction between the constituents of the Chaga mushroom with the constituents of the esterification mixture or Manuka honey, and combining that heterogenous mixture with a medium chain triglyceride.

The type of reaction most likely induced first is known as a Maillard reaction, also called a non-enzymatic browning reaction and is responsible for the formation of intermediates that would typically form a known end-product but are redirected into a novel reaction with betulinic acid and possibly other constituents found in the Chaga mushroom reductions and extracts to form novel compounds, as described herein. The Maillard reaction starts with a reaction between a reducing sugar and various types of amino acids when certain reaction parameter conditions are met. Two of the several intermediate products formed during the beginning of this reaction are known as Amadori and Heyns products. The full elucidation of a Maillard reaction remains unknown, but the various possible outcomes have been studied, as described herein, and measurements confirming the formation of these products have been achieved, as also described herein.

Another embodiment is a chemical compound. The chemical compound is isolated from the medicinal formulation or synthesized and is optionally combined with a pharmaceutically-acceptable carrier to form a pharmaceutical composition.

Another embodiment is a method of treating cancer by administering one or more of the medicinal formulations, chemical compounds, or pharmaceutical compositions to a human or animal.

### Medicinal Formulation

The medicinal formulations provided herein are based on Chaga mushrooms, in some formulations along with honey, in particular Manuka honey, and in some formulations medium chain triglyceride oil, and their administration to mammalian patients suffering from various cancers. Mammalian patients include human patients and animal patients.

While the abilities of many diverse types of mushrooms to affect the course of a cancer have been examined, none have proven to be particularly successful, either as a primary therapy or as an adjunct therapy, with most mushroom-based formulations stalling out in the laboratory. In the *in vitro* studies discussed herein, the typical method of preparation of these Chaga extracts were measured against the novel Chaga formulation. The Chaga formulation outperformed the other common methods of preparation.

One approach to expanding the potential usefulness of mushrooms in combating the effects of cancer is to combine the mushroom with another ingredient. There is particular interest in exploring a combination of the Chaga mushroom with a honey, in particular Manuka honey as well as medium chain triglyceride oil. In some embodiments, a formulation uses a Chaga extract and a Chaga reduction mixed with Mauka honey. In some embodiments the Chaga extract and the Chaga reduction may be distilled before addition of the honey. The formulation may be administered orally. In other embodiments, particularly where the formulation is to be administered topically, the formulation may be mixed with coconut oil or an alternative medium chain glyceride.

### Chaga Mushroom

The compound of greatest interest that can be extracted from the Chaga mushroom is betulinic acid (also referred to herein as "BA"). Betulinic acid is a pentacyclic triterpene, recently shown to have anti-cancer properties, but lacking in any clinical results. Betulinic acid on its own has limited bioavailability, having a molar mass of 456.7 g/mol and boiling point of 550 °C. Past studies of betulinic acid have pointed to potential limiting factors in its usage, including its molecular size, poor aqueous solubility and low bioavailability. In its raw organic form in the Chaga mushroom, betulinic acid is bound to chitin, the primary component of cell walls in fungi. Extraction of betulinic acid from the Chaga mushroom, therefore, must include releasing the betulinic acid from its chitin binding.

Betulinic acid has been the subject of numerous studies and has been shown to induce apoptosis and to defragment DNA by inhibiting topoisomerase in the mitochondria of cancer cells. There has also been a suggestion that betulinic acid has an ability to reverse the Warburg effect, a form of modified metabolism found in cancer cells which favor a specialized fermentation of the aerobic respiration pathway preferred by most other cells of the body. In this fermentation process, the last product of glycolysis, pyruvate, is converted into lactate or ethanol, while yielding lower amounts of ATP than in the citric acid cycle. However, it allows cancer cells to convert glucose and glutamine into biomass by avoiding catabolic oxidation into carbon dioxide, thus preserving carbon-carbon bonds, and promoting anabolism. The mechanism by which betulinic acid can interrupt the Warburg effect is currently unknown, although the induction of cellular respiration and reversal of the fermentation process of glucose, the cancer cells' main pathway of utilizing glucose, could be a meaningful adjunct process for combatting the growth of cancer cells.

In addition to these potential functions, betulinic acid along with other bioactive compounds found in the Chaga mushroom are known to break down lactate at high rates. The removal of lactate can assist with combating the acidic, immune system-hostile, environment produced by cancer cells: the relatively high quantity of lactic acid creates a more acidic environment for the cancer cells, which favor a lower pH level than healthy cells. Some literature suggests that the lactic acid may also assist in providing for cancer cells to avoid detection by the immune system, allowing them to evade destruction by the immune cells. There are specific immune cells that when activated can detect and destroy cancer cells. Some of these types of cells are T-cells, specifically programmed to destroy cancer cells. When these immune cells can identify cancerous cells, they are quite capable of destroying the cancer cells.

It has also been suggested that betulinic acid may preferably create an oxidative stress load in cancer cells, and that this stress load may be a factor in how betulinic acid inhibits topoisomerase, and thus inhibiting DNA production, while seeming to be harmless to the surrounding healthy cells.

Also of interest is the high level of superoxide dismutase (SOD) in both Chaga extract and Chaga reduction. SOD's may be useful in dismutating the reactive oxygen species (ROSs) produced by the cancer cell's preferred mode of action and a part of the cellular damage cycle that induces signaling possibly linked to the spread of metastatic disease. Normal healthy cells produce their own SODs to manage oxidative stress from radical oxygen species to maintain cellular health, but cancer cells become unable to manage cellular damage, and lose the ability to signal apoptosis due to cellular damage. The high content of SODs provided in the formulation may be of assistance to particular cancer cells in repairing damage and establishing a normal metabolic function once again.

There are potentially additional compounds present in Chaga that can benefit the host's immune cell's ability to counter-act cancer progression.

### Manuka Honey

Manuka honey is a monofloral or multifloral honey produced from nectar of the manuka plant *(leptospermum scoparium),* which is native to south-east Australia and New Zealand.

A test for monofloral manuka honey, adopted by the New Zealand Ministry for Primary Industries (see https://www.mpi.govt.nz/food-business/honey-bee-products-processing- requirements/manuka-honey-testing/) is that the honey has the five following characteristics:
- contains 3-phenyllactic acid at a level greater than or equal to 400 mg/kg;
- contains 2'-methoxyacetophenone at a level greater than or equal to 5 mg/kg;
- contains 2-methoxybenzoic acid at a level greater than or equal to 1 mg/kg;
- contains 4-hydroxyphenllactic acid at a level greater than or equal to 1 mg/kg;
   and
- DNA level from manuka pollen is less than Cq36, which is approximately 3 fg/µL.

A test for multifloral Manuka honey, also adopted by the New Zealand Ministry for Primary Industries, is that the honey has the five following characteristics:
- contains 3-phenyllactic acid at a level greater than or equal to 20 mg/kg but less than 400 mg/kg;
- contains 2' -methoxyacetophenone at a level greater than or equal to 1 mg/kg;
- contains 2-methoxybenzoic acid at a level greater than or equal to 1 mg/kg;
- contains 4-hydroxyphenllactic acid at a level greater than or equal to 1 mg/kg;
   and
- DNA level from manuka pollen is less than Cq36, which is approximately 3 fg/µL.

When the term "Manuka honey" is used herein, it refers to a honey that passes at least the DNA test (wherein the DNA level from manuka pollen is less than Cq36, which is approximately 3 fg/µL) for multifloral Manuka honey or for monofloral Manuka honey, as **set forth** in the previous two paragraphs.

Although not wishing to be bound by the following, several hypothetical reactions and mechanisms of action are described as follows. Manuka honey may be symbiotically contributing to the formula's function. One particularly interesting finding is that Manuka contains a high content of methylglyoxal, which is known to be produced in organisms as a side-product of several metabolic pathways, mainly glycolysis. While endogenous methylglyoxal in animals has been attributed to the formation of advanced glycation end products (or AGEs), which are used as biomarkers in aging and in the development of many degenerative diseases, research suggests that methylglyoxal derived from honey, such as Manuka, does not cause an increase in **advanced** glycation end products in healthy persons. Furthermore, methylglyoxal in Manuka has been shown to have antibacterial activity against *E. coli* and S. *aureus.* attributed it by living cells as a sort of "self-defense" mechanism. This may give the potential for methylglyoxal to make the cancer cells more susceptible to the functions of betulinic acid, along with other compounds found in both Manuka honey and Chaga mushroom.

Manuka honey, as well as other honeys, contains high concentrations of fructose and amino acids, among other molecular compounds formed when the honey is produced. Fructose being found in high concentration, is one of a few types of reducing sugars. Many different amino acids are also found in Manuka and other types of honey in varying concentrations. The ability for fructose and amino acids to react together in an induced reaction has been shown. This type of reaction requires certain parameters and would not spontaneously occur outside of these reaction parameters. This type of documented reaction is known as a Maillard reaction and occurs when a condensation reaction is created between a reducing sugar and an amino acid. Both fructose and glucose are capable of functioning as a reducing sugar during a Maillard reaction with amino acids, undergoing a condensation reaction first to form intermediate products known as Amadori and Heyns products which are two examples of several types of intermediate products formed during a Maillard reaction. During the formation of these intermediate products, methylglyoxal is one of the reactive intermediates that assists in the formation of the final product outcomes of a typical Maillard reaction. The formation of a novel product with a fructose-amine-triterpene, or other constituent, lends to a function of interacting with cancer cells potentially through glucose receptors as well as potentially other cell signal receptors. This possible novel product likely retains a function-structure similar to that observed in other glucoside like therapeutics both found in nature and produced in pharmaceutical labs. A hypothesis proposed here is that the possible novel product could have a similar structure-function and therefore be readily consumed by the cancer cell, thus making betulinic acid and other compounds more bioavailable.

The antimicrobial properties of Manuka honey may also play a role in limiting proliferation of microbes that could be inhibitory to the immune system's destruction of the cancer cells. There is limited understanding of how certain microbes can be a catalyst in the synthesis of betulinic acid during the growing phases of wild Chaga mushroom, and is also found in conversion attempts from betulin to betulinic acid in various lab work. Thus, the provision of the "raw materials" via the Chaga extract, in addition to the "blueprint" for betulinic acid with the Chaga mushroom extract, may result in the immune system's own microflora assisting or even accelerating this process of synthesizing higher content of betulinic acid and utilizing this process against cancer cells. This process can be hindered by opportunistic microbes, and interestingly some of those microbe species such as members of the *Enterbacter* family such as *H.Pylori* have been listed as a carcinogen in humans. It is postulated here that the antimicrobial functions of Manuka also help to create a friendly microbial environment that better facilitates the synthesis and reaction of betulinic acid and other compounds. This function may also facilitate the increasing bioactivity of betulinic acid, and allow for better utilization of betulinic acid and other compounds by healthy immune cells.

Another property of Manuka honey is its potent anti-biofilm properties, which may be important due to the nature of bio-film creation by hostile microbes. One of the many, not fully understood, actions against bio-films is to reverse genetic mutations in microbes that have become resistant to antimicrobial treatments, which makes them more easily eradicated by Manuka's own antimicrobial function. Bee defensin is prevalent in all honeys, but the Manuka honey displays some unique protective functions that are not peroxidase based. This may be important in that the Manuka honey compounds do not destroy friendly microbes that could be utilized in synthesis actions as well as protecting healthy cells.

Manuka honey has a relatively low pH, about 3.5-4.5, which contrasts with the higher pH of the Chaga extract and reduction. Furthermore, the lower pH of the honey may contribute to the formula's action on cancer cells because **it may be less** affected by one of the cancer cell's primary defense mechanisms, i.e., the reduction of pH levels to evade immune cell attacks. This also may permit Manuka to inhibit microbial growth, stimulate the bactericidal actions of macrophages in the host's immune system and, in chronic wounds, to reduce protease activity and increase fibroblast activity and oxygenation.

In addition to the earlier functions discussed, recent studies have demonstrated that Manuka honey can exert anti-proliferative effects against cancer cells. These anticancer properties can involve different processes, including inducing apoptosis in cancer cells through the depolarization of the mitochondrial membrane, inhibiting cyclooxygenase-2 by various constituents (like flavonoids), releasing cytotoxic H2O2, and scavenging of reactive oxygen species (or ROSs). The main mechanism by which Manuka exerts its anti-proliferative effect is through the activation of mitochondrial apoptotic pathways, involving the stimulation of the initiator, caspase-9, which determines the activation of the executioner, caspase-3. This last function is also shared by Chaga mushroom. Moreover, it can induce apoptosis via the activation of PARP, the induction of DNA fragmentation and the loss of Bcl-2 expression.

*In vivo,* Manuka honey has been shown to be effective in decreasing tumor volume and supporting apoptosis of tumor cells in a mouse melanoma model, reducing colonic inflammation **in** inflammatory bowel disease in rats, restoring lipid peroxidation and improving antioxidant parameters. Studies show Manuka honey has no detrimental effect in relation to advanced glycation end products nor to a change in gut microbiota homeostasis. This is important as it relates to the unknown properties of how methylglyoxal relates to the progression of certain chronic diseases.

### Carrier

Coconut oil or an alternative medium chain triglyceride may be used to carry the formulation and contribute biochemically to its function, particularly where the formulation is applied topically. The medium chain triglyceride adds a protective layer when the formulation is applied to an open skin lesion as well as subdermal tumors. The carrier is preferably a soft solid, that can be applied as a paste, or a liquid, and can preferably dissolve the Chaga extraction/reduction as well as the honey. In some embodiments the carrier/formulation mixture may be sprayed on to the area to be treated. Coconut oil is a white, solid fat that melts at a temperature of approximately 25°C to make a clear thin liquid oil. Alternative medium chain triglycerides, for example palm oil, may be liquid at room temperature.

### Preparation of a Chaga extraction

An extraction of Chaga mushrooms may be made using the following approach. The Chaga mushroom is chopped to increase the surface area exposed to liquid. In some embodiments, the Chaga is chopped so that 90% of the granules have a maximum dimension of less than 7 mm in maximum dimension, preferably less than about 5 mm, more preferably less than about 3 mm and more preferably less than about 2 mm.

The chopped Chaga is then mixed with an extraction solvent. The extraction solvent typically includes a nonaqueous solvent and may be a solvent typically used for food extraction. Examples of nonaqueous solvents include a short chain alcohol such as ethanol, a short chain glycol such as propylene glycol, a short chain acid, such as methanoic acid, ethanoic acid or lactic acid, a short chain ketone such as acetone, or a short chain ester such as ethyl acetate or n-butyl acetate. If a solvent is used that would be toxic to the patient, it can be removed using standard chemical processing means. The extraction solvent may be present in an amount such that the dry volume of chopped Chaga is about one half the volume of the extraction solvent, although more or less may be used.

Water may be included in the extraction solvent, with the ratio of non-aqueous solvent to water in the extraction solvent being between about 5:95 to I 00:0. Preferably the ratio of non-aqueous solvent to water is in the range 95:5 to 60:40, more preferably in the range 90: 10 to 70:30. In other words, the Chaga is mixed into a liquid that includes at least one non-aqueous solvent. The extraction solvent dissolves components of the Chaga released from the mushroom into the solvent. The non-aqueous solvent tends to dissolve the more lipophilic components of the Chaga while the water tends to dissolve the less lipophilic components of the Chaga, since the water is commonly more polar than the non-aqueous solvent. It will be appreciated that other non-aqueous solvents may be used.

The dielectric constant, ε , of the extraction solvent is preferably less than 50, more preferably less than 40 and may even be less than 30. For example, where the liquid solvent is 100% ethanol, the dielectric constant, ε ethanol, is approximately 24.5. In a mixture of miscible liquids, the dielectric constant may be calculated by taking a volumetric average of the dielectric constants. For example, in a liquid containing 90% ethanol by volume and 10% water (ε water = 80.1) by volume, the dielectric constant of the mixture is given by (0.9 x ε ethanol) + (0.1 x ε water) = 30.2. The dielectric constant of the liquid is an indicator of the polarity of the solvent mixture.

Larger values indicate that the solvent polarity is higher, which may result in the extraction of less lipophilic constituents of the Chaga mushroom. Lower values of dielectric constant indicate that the solvent polarity is lower, in which case the solvent may be more effective at extracting more lipophilic components from the Chaga mushroom. The solvents used may be either protic or nonprotic. A mixture of ethanol and water is a mixture of two protic solvents.

The Chaga/liquid mixture is then left for a predetermined time suitable for components of the mushroom to be extracted. In some embodiments, the mixture is left at room temperature for more than 48 hours, more than a week, preferably more than a month, more preferably more than two months and even more preferably more than three months. The mixture may be covered or sealed to prevent evaporation of the liquid components. The extraction time may be shorter if the mixture is held at an elevated temperature, for example 90 °F, 100 °F, or higher, for example up to about 160 °F and/or if the mixture is agitated, for example using a magnetic stirrer. The temperature of the mixture is kept below the boiling point of the liquid.

Another approach to forming an extraction is to use an ultrasonic extraction method, in which ultrasound waves are introduced to the Chaga/liquid mixture. Ultrasonic waves, which may be generated by an ultrasonic probe or other suitable ultrasonic generator travel through the liquid creating alternating high-pressure/low-pressure areas, which can result in acoustic cavitation. This, in tum, can result in locally extreme temperatures and pressures, heating/cooling rates, pressure differentials and high shear forces. When cavitation bubbles implode on the surfaces of the Chaga parts, mass transfer from the Chaga parts into the liquid is enhanced. Under ultrasonic extraction, the Chaga/liquid mixture is exposed to ultrasonic waves for a predetermined period of time, typically a few minutes to hours, to transfer components from the Chaga into the liquid. Ultrasonic extraction may take place at room temperature or at elevated temperatures.

The mixture is then filtered to remove the solids. Any suitable method of filtering may be used, depending on how the liquid extraction is to be used. For example, if the liquid extraction is to be taken orally or applied topically, then the presence of some small Chaga particles, typically < 1 mm, may be acceptable, and a filter as coarse as a tea strainer may be acceptable. If, on the other hand, the extract is to be sprayed onto the recipient, then particles as large as **1** mm may block the spray equipment and a finer method of filtering, for example using a filter paper may be used. The extraction is preferably stored in an acid resistant container, for example a glass container.

The Chaga extract typically has a pH level ranging from 4-9. Generally, the pH of the Chaga extract is lower with longer extraction times and with less polar liquids.

### Method of Preparing a Chaga reduction

A reduction of Chaga mushrooms is made by immersing the chopped Chaga in water, at an elevated temperature for a period of time, or cycled through elevated temperatures for a number of times. In one approach, the Chaga parts are exposed to a boiling or greatly elevated temperature. The Chaga mushroom is chopped so that 90% of the granules have a maximum dimension of less than about 75 mm in maximum dimension, preferably less than about 50 mm, more preferably less than about 30 mm and more preferably less than about 20 mm. The Chaga may also be chopped as finely as discussed above with respect to the extraction, e.g., so that 90% of the granules have a maximum dimension of less than 7 mm in maximum dimension, less than about 5 mm, less than about 3 mm and even less than about 2 mm. The Chaga reduction is made using a solvent that contains at least water and, optionally another liquid, although the solvent used in a reduction is more polar than that used in an extraction.

In one approach, the chopped Chaga is placed in a reduction solvent such as water, preferably in a volume ratio of about 1:40 to about I :2. Preferably the chopped Chaga is covered by the reduction solvent. The reduction solvent may be brought to a boil and then allowed to cool down again in a cycle. During the boiling/cooling cycle, the reduction solvent is brought to a strong boil for a time and kept at an elevated temperature for a time before being allowed to cool off. In some embodiments, the reduction solvent is brought to a strong boil for 1-10 minutes, more preferably 4-5 minutes, and then brought to a simmering boil for about 10-60 minutes, preferably 15-45 minutes, more preferably 20-30 minutes, with the remainder of the cycle permitting the water to cool. An exemplary cycle includes heating to a boil, achieving a rolling boiling for about 4-5 minutes, reducing to a simmering boil for about 20-30 minutes, and cooling for the rest of the cycle. The cycle may take, for example, one hour or more.

In another approach to forming a reduction, the chopped Chaga is heated to an elevated, but not boiling, temperature, for example 160 °F or higher, for a period of time, for example 24 - 96 hours, 3 - 7 days, or even longer.

The reduction solvent may include a mixture of different liquid solvents. The dielectric constant, ε, of the reduction solvent is preferably more than 50, more preferably more than 60 and may even be more than 70. For example, where the liquid solvent is 100% water, the dielectric constant, ε water, is approximately 80.1. Here, values of dielectric constant are provided as the d.c. dielectric constant. In a mixture of miscible liquids, the dielectric constant may be calculated by taking a volumetric average of the dielectric constants. For example, in a liquid containing 90% water by volume and 10% ethanol by volume, the dielectric constant of the mixture is given by (0.9 x ε water) + (0.1 x ε ethanol) 74.5. The dielectric constant of the reduction solvent is greater than the dielectric constant of the extraction solvent, for example, by more than 10, more than 20, or even by more than 30. In an example where an extraction solvent is 9:1 parts ethanol to water by volume, the dielectric constant is about 30.2, whereas in in a reduction solvent containing 9:1 parts water to ethanol by volume, the dielectric constant is about 74.5, a difference of about 34.

The mixture is then filtered to remove the solids. Filtering may be performed after allowing the water to cool. Any suitable method of filtering may be used, depending on how the liquid reduction is to be used. For example, if the liquid reduction is to be taken orally or applied topically, then the presence of some small particles, typically< 1 mm, may be acceptable. If, on the other hand, the reduction is to be sprayed onto the recipient, then particles as large as 1 mm may block the spray equipment and a finer method of filtering, for example using filter paper, may be used.

In some approaches, the reduction is formed using a mixture of water and some other, nonaqueous, solvent, such as a food grade solvent. For example a nonaqueous solvent may be a short chain alcohol such as ethanol, a short chain glycol such as propylene glycol, a short chain acid, such as methanoic acid, ethanoic acid or lactic acid, a short chain ketone such as acetone, or a short chain ester such as ethyl acetate or n-butyl acetate If a solvent is used that would be toxic to the patient, it can be removed using standard chemical processing means.

### Method of Preparing an extraction/reduction mixture

The extraction may be mixed with the reduction to produce an extraction/reduction mixture (ER mixture). The ratios of volumes of the extraction and reduction used to form the ER mixture may cover a wide range, for ex<:1mple from 50:1 (i.e. 10 parts extraction to 1 part reduction) to 1:50 (i.e. **1** part extraction to 50 parts reduction), preferably from 5:1 to 1:20, more preferably 1:1 to I: 10 and even more preferably 1:3 to 1:7. The volume ratio of extraction and reduction in ER mixture may be around 1:5. In some approaches, the extraction is added to the reduction in an acid-resistant container, for example a glass container.

### Proposed Reaction Mechanism

Although not wishing to be bound by the following proposed reaction mechanism theory, the Maillard reaction occurring in the method of preparing the Extraction/Reduction mixture described above most likely induces a condensation reaction. This might start with fructose and proline, an amino acid in high concentration in honey, and the product of that reaction reacts with methylglyoxal/betulinic acid, since methylglyoxal is a precursor to protein glycation. The condensation reaction with betulinic acid/ methylglyoxal and then one or more of the fatty acids could also be set in motion during certain phases of the Maillard reaction when some of the intermediates are very reactive. If this is happening, it could be creating a glycoside-like molecule from the fructose/proline/methylglyoxal/betulinic acid with a fatty acid component. If possible, this could help explain the non-polar/polar indications found in the formula as some of the pH tests show various pH levels in the final assembly. This could also explain the fluorescent nature of the formula found in the first *in vitro* studies, the final measurements had to be done using an alternative to the fluorescence measurements because of absorbance. If the resulting active ingredient has a glycoside quality, and it survived the digestive system intact, it could be attractive to the glucose receptors on cancer cells and promote binding.

Additional theories for this reaction mechanism are that the Maillard reaction is initiated at the temperature range in which the above-described reaction mixture is incubated; the Maillard reaction has the capacity to induce a condensation reaction, especially if fructose is involved, as it reacts with available carbonyl groups; a reaction between fructose and proline is highly likely, and the product of that reaction is commercially available from Toronto Research Chemicals; the reaction could also involve glycine, or other amino acids; the Maillard reaction could protect the molecule from breaking down in the stomach and small intestine, making it possible to reach the cancer cells while intact; the combination of polar and non-polar starting materials reacting might facilitate a spherical structure, a structure that has been thought to be observed in some of lab tests of substances provided herein; if the structure of the molecule created is a micelle-like structure, that could explain the hydrophobic/hydrophilic characteristics observed; a micelle structure could facilitate transport through the bloodstream in or around the small intestine and is feasible given the observed particle size, it seems feasible.

### Method of Producing a Medicinal Formulation

Formulations may be formed using any combination of the products listed above, including the Chaga extraction, the Chaga reduction, the distillate, and the remaining fraction. In addition, the formulation may include a honey such as a manuka honey, and a carrier, such as coconut oil or other medium chain triglyceride. For example, an oral formula may include the Chaga extraction, the Chaga reduction and the Chaga distillate in a suitable ratio. Typically, the reduction is present in an oral formula at a greater amount than the Chaga extract or the distillate, although this is not a necessary condition. For example, in the formulation, the ratio of reduction to extract may lie in the range of 100:1 to 1:100, preferably in the range 20:1 to 1:10 and more preferably in the range 20:1 to 1:1. The ratio of reduction: remaining fraction may also lie in the range of 100:1 to 1 :100, preferably in the range 20:1 to 1:10 and more preferably in the range 20: 1 to 1: 1. The ratio of the extract: remaining fraction may lie in the range of 100: 1 to 1:100, preferably in the range 1 0:1 to 1:10 and more preferably in the range 5:1 to 1:5. In one particular example, the weight ratio of the Chaga reduction, Chaga extract and distillate is 5:1:1. Other ratios may be used within the range limits discussed.

Additionally, if administered orally, the formulation may also include manuka honey typically, but not necessarily, in an amount less than the amount of distillate or extract. For example, the weight ratio of Chaga reduction, Chaga extract, distillate and manuka honey in a formulation may be 10:2:2:1. Other ratios may be used. In particular, the weight ratio of reduction: honey may lie in the range 10:0 to 10:10, or in the range 10:0.1 to 10:10, although ratios higher than about 10:2 may change the consistency of the formulation and also become expensive.

The formulation may also include a carrier, such as coconut oil or other medium chain triglyceride typically, but not necessarily, in an amount less than that of the distillate. The weight ratio of reduction: carrier may lie in the range 10:0 to 10:50, or 10:0.1 to 1:50, depending on the method in which the formulation is to be administered. For example, in a liquid formulation to be applied to a horse's feed, the weight ratio of reduction: carrier may be l 0:0 to about 10:2. In one example of a liquid formulation, the weight ratio of Chaga reduction, Chaga extract, distillate and coconut oil in the formulation may be 10:2:2:1. Higher amounts of carrier may be used for a more solid formulation, such as a salve. In an example, the reduction: carrier weight ratio may be 10: 10. Other ratios may be used.

The formulation may include both manuka honey and a carrier such as coconut oil or other medium chain triglyceride. The amount of each of the manuka honey and the carrier may be, but is not required to be, less than that of the extract and distillate. For example, the weight ratio of Chaga reduction, Chaga extract, distillate, manuka honey, and coconut oil in a formulation may be 10:2:2: l: 1. Other ratios may be used.

An alternative formulation is provided, which is a reaction between the Chaga extract described above with Proline, Fructose and a fatty acid such as medium chain triglyceride oil or Sunflower lecithin, or other surfactant, for the esterification process of betulinic acid with fructose and proline to create a fructose-amine-triterpene. These are the isolated starting materials originally supplied by the Manuka Honey. The fully synthesized product is suspended in the original formulation, and all material is food grade and FDA approved for sale in original form.

### Administration of Medicinal Formulation

The medicinal formulation may be administered in any suitable manner known to those skilled in the art. The medicinal formulation is a liquid and may be administered orally or applied to solid food. For example, the formulation may be orally consumed by the patient or with the formulation may contain additives, such as sugar, salt, etc., that may be used to alter the flavor to a taste preferred by the patient. In other approaches, the formulation may be added to another liquid to be drunk by the patient. For example, in the case of a human patient, the formulation may be added to a drink such as coffee, tea, a carbonated soda or the like. In the case of an animal patient, such as an equine patient, the medicinal formulation may be added to the animal's water. In other approaches, the formulation may be mixed together with food. For example, in the case of a human patient, the formulation may be added to soup, included in gravy served over meat, vegetables or potatoes, or included in a sauce served with, e.g. pasta or meat. In the case of an animal patient, such as an equine patient, the formulation may be fed directly or may be sprinkled over the horse's feed. When fed directly, an additive may be included to mask the taste of the formulation. One example of a taste-masking agent is sugar. The formulation is well absorbed in pelleted feed. Whole grain feeds and hay, on the other hand do not absorb the formulation as well, in which case the formulation may include a coating agent such as medium chain triglyceride oil so that the formulation adheres to the feed.

In other approaches, the formulation may be applied topically. In some cases, the formulation may be made more viscous when applied topically, for example by adding a more viscous carrier such as coconut or other medium chain triglyceride oil, or a thicker oil. The formulation may be applied topically using a towel, piece of cloth, or pad soaked in the formulation. Another approach is to apply a pad, poultice or the like, that has been soaked in the formulation, to the area to be treated and to hold the pad, poultice or the like in place against the skin. The pad, poultice or the like may be held in place using, for example, adhesive strips, a bandage or any other suitable method. Where the formulation includes a relatively large fraction of carrier, the formulation may have the consistency of a salve that can be spread on the area of concern.

In other approaches, the formulation may be included in a gel, paste or lotion that may be applied to the area to be treated.

In other approaches, the formulation may be sprayed on the area to be treated. In such a case, it may be preferred to include the formulation with a viscous carrier, and for the formulation to be more finely filtered than needed for oral administration, in order to prevent clogging the spraying equipment.

Additional methods of administering the medicinal formulation are provided below with respect to administration of the pharmaceutical compositions containing the isolated or synthesized chemical compound represented by Formula 1 and Formula 2.

The medicinal formulation may be administered to a patient at suitable dose levels. For example, an oral formulation may be dosed daily at between 0.1 ounce per 1000 lb. (approximately 0.065 mL/kg) of patient weight and 1 ounce per I lb. (approximately 6.5 mL/kg) of patient weight. In some embodiments, the medicinal formulation is administered at a daily dosage of 1 ounce per 100 lb. of patient weight (approximately 0.65 mL/kg). The medicinal formulation may be provided in a single daily dose or in two or more smaller doses in a day.

The medicinal formulation may be administered with other therapies. For example, a patient taking the medicinal formulation as described herein may also be on a standard antibiotic regimen.

### Chemical Compounds and Methods of Production

Novel chemical compounds are provided herein. The novel compounds are isolated from the medicinal formulation or are synthesized. The following chemical compounds successfully demonstrate anti-cancer activity *in vitro.*

In one embodiment, the chemical compound is represented by Formula 1, below. 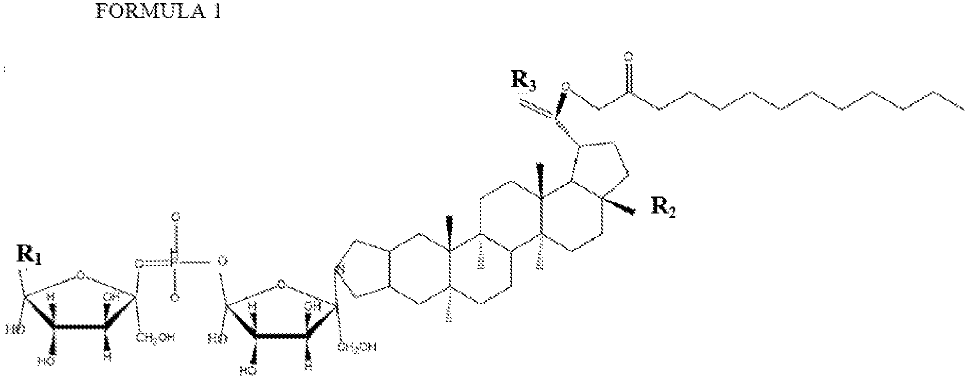 wherein R₁-R₃ are, independently, alkyl/alkane, alkene/alkenyl, or alkyne/alkynyl, having from 1 to 20 carbon atom; benzene/aromatic/phenyl, ether, amide, alkyl halide, amine (-amino), alcohol/hydroxy/hydroxyl (-OH), thiol, aldehyde, ketone, ester/ester quat, carboxylic acid (COOH), acid anhydride/acetic anhydride, acyl halide, or methyl.

In another embodiment, the chemical compound is represented by Formula 1, below. 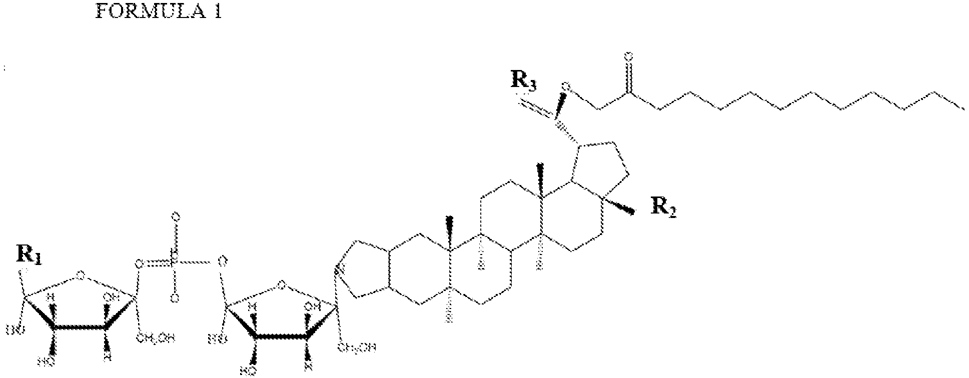 wherein R₁-R₃ are, independently, alkyl/alkane, alkene/alkenyl, or alkyne/alkynyl, having from 1 to 20 carbon atom; benzene/aromatic/phenyl, ether, amide, amine (-amino), alcohol/hydroxy/hydroxyl (-OH), thiol, aldehyde, ketone, ester/ester quat, carboxylic acid (COOH), acid anhydride/acetic anhydride, or methyl.

In another embodiment, the chemical compound is represented by Formula 1, below. 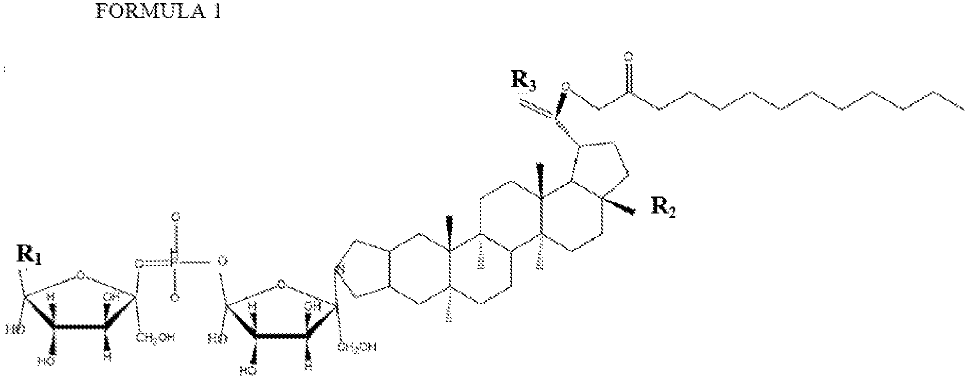 wherein R₁-R₃ are hydroxyl.

In another embodiment, the chemical compound is represented by Formula 2, below. 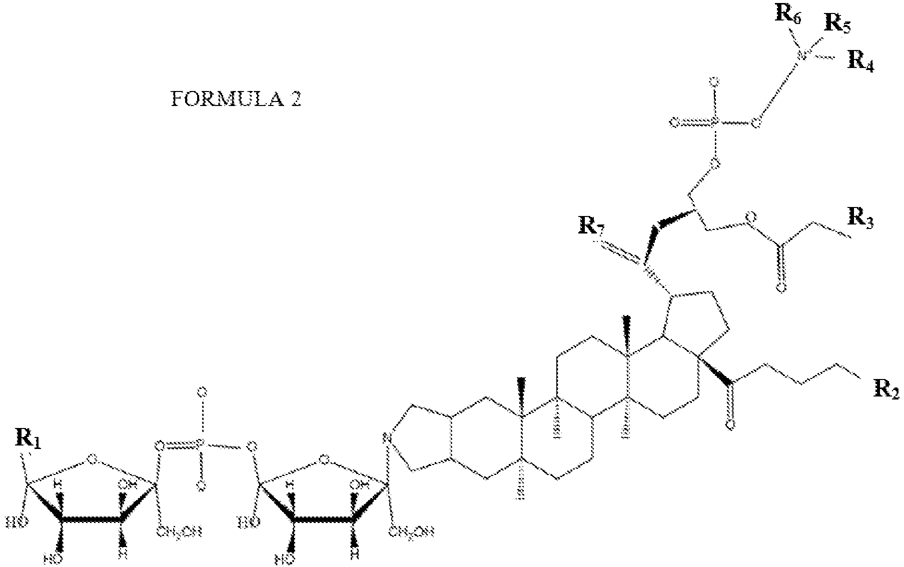 wherein R₁-R₇ are, independently, alkyl/alkane, alkene/alkenyl, or alkyne/alkynyl, having from 1 to 20 carbon atom; benzene/aromatic/phenyl, ether, amide, alkyl halide, amine (-amino), alcohol/hydroxy/hydroxyl (-OH), thiol, aldehyde, ketone, ester/ester quat, carboxylic acid (COOH), acid anhydride/acetic anhydride, acyl halide, or methyl.

In another embodiment, the chemical compound is represented by Formula 2, below. 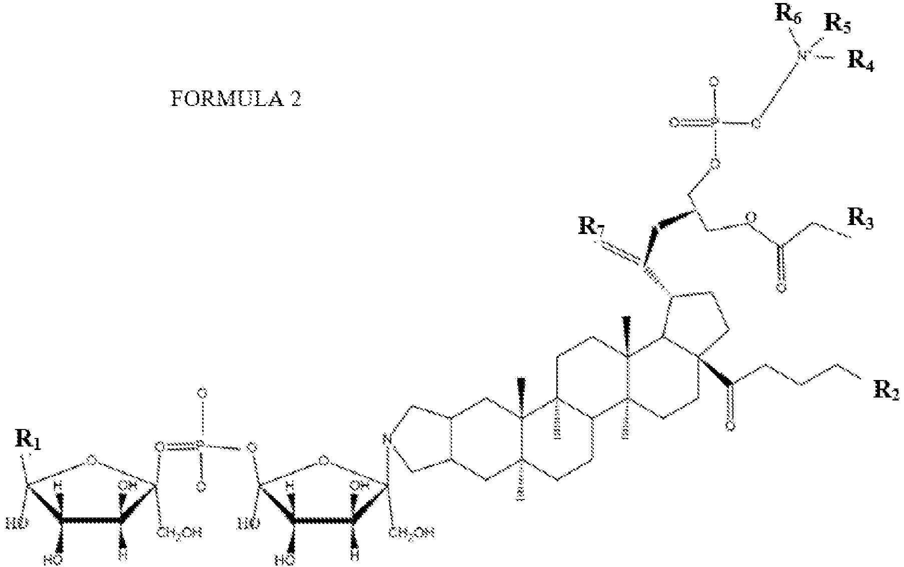 wherein R₁-R₇ are, independently, alkyl/alkane, alkene/alkenyl, or alkyne/alkynyl, having from 1 to 20 carbon atom; benzene/aromatic/phenyl, ether, amide, amine (-amino), alcohol/hydroxy/hydroxyl (-OH), thiol, aldehyde, ketone, ester/ester quat, carboxylic acid (COOH), acid anhydride/acetic anhydride, or methyl.

In another embodiment, the chemical compound is represented by Formula 2. 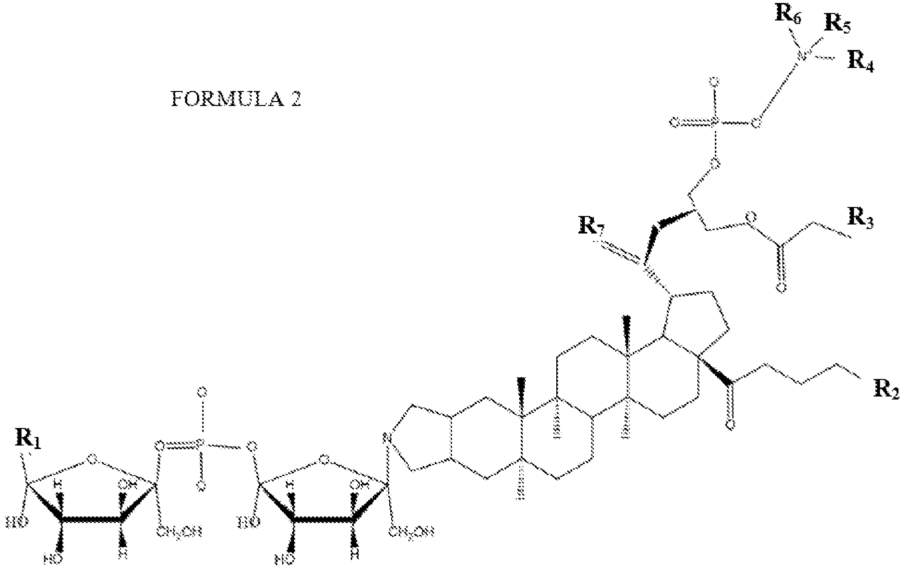 wherein R₁-R₇ are, independently, hydroxyl.

The chemical compounds provided above are isolated from the medicinal formulation as described in more detail in the Examples.

Confirmation of the isolate or synthetic compound as a betulinic acid derivative is determined by analytical techniques such as HPLC, GC. Mass Spectrometry or NMR.

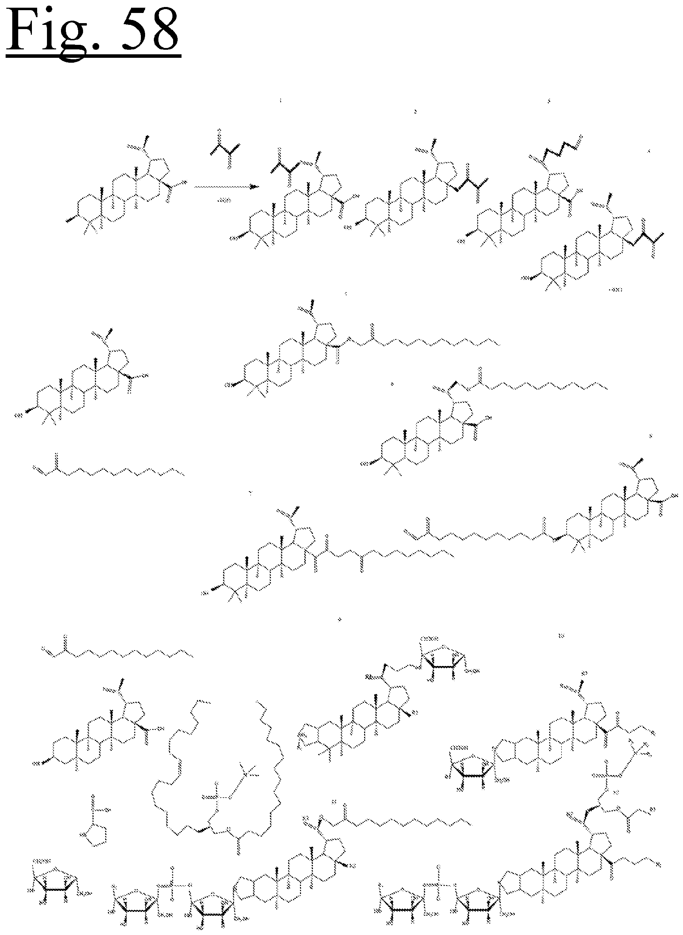

The chemical compounds are synthesized using the reaction pathways shown in Fig. 58. The compound represented by Formula 1 was synthesized using a reaction temperature from 240 to 260°C. The compound represented by Formula 2 was synthesized using a reaction temperature from 240 to 260°C.

### Pharmaceutical Compositions and Methods of Production

The pharmaceutical composition provided herein contains one or more of the chemical compounds described above in combination with a suitable carrier. The pharmaceutical composition is produced by combining or mixing one or more of the chemical compounds provided above with a pharmaceutically acceptable carrier in accordance with methods known to those skilled in the art.

Suitable carriers include artificial and biological delivery systems such as, but not limited to liquids, gels, suspensions, emulsions, dendrimers, quantum dots, hydrogels, aerogels, foams and creams. Suitable carriers also include nano drug delivery carriers such as, but not limited to, nanospheres, hydrogels with and without nanoparticles, nanocapsules, nanotubes, and nanoparticles. Microsystems are also suitable carriers such as, but not limited to, partches and micropumps. Vesicles of biological or inert origin are also included in the list of suitable carriers, such as, but not limited to, liposomes, aquasomes, niosomes, ethosomes, polymersomes and cubosomes. In addition, carriers having a biological origin such that they provide a class of macromolecules include, but are not limited to, lipids, carbohydrate structures, proteins, peptides and nucleic acids.

### Administration of Pharmaceutical Compositions

The pharmaceutical composition may be administered in any suitable manner known to those skilled in the art. Suitable delivery systems include passive delivery systems, such drug delivery via diffusion, or active delivery systems, such as drug delivery via digestion. The pharmaceutical composition may be administered orally, in liquid form or in the form of a solid, such as a pill, capsule or powder; intravenously, topically, subcutaneously, vaginally or rectally, such as with a medicated suppository; via ocular means, such as eye drops, ointments or medicated contact lenses; via transdermal means, such as a patch, via pulmonary means, such as an inhaler, via microelectrochemical systems, or via micropumps. With the potential for nanoparticle carriers, many options for administration exist and are known to those skilled in the art.

Oral formulations of the pharmaceutical composition may be administered to a patient at suitable dose levels calculated to achieve the desired chemotherapeutic effect. In one embodiment, an oral formulation having a 10-1000 ug/mL concentration of the compound of Formula 1 or 2 in an aqueous solution may be dosed daily at between 0.1 ounce per 1000 pounds body weight (approximately 0.065 mL/kg of patient weight) and 1 ounce per I pound (approximately 6.5 mL/kg of patient weight). In some embodiments, the oral formulation is administered at a daily dosage of 1 ounce per 100 lb. of patient weight (approximately 0.65 mL/kg). The oral formulation may be provided in a single daily dose or in two or more smaller doses in a day. If the pharmaceutical composition exhibits increased potency, the pharmaceutical dose is reduced accordingly. In another embodiment, the dose of the pharmaceutical composition is a range of from 0.001 mL/100 lbs. body weight to 3 ml/100 lbs. body weight. In another embodiment, the dose of the pharmaceutical composition is 0.03 mL per 100 pounds of body weight.

The pharmaceutical composition may be administered with other therapies. For example, the pharmaceutical composition may be administered to a patient simultaneously or sequentially with a second therapeutic agent, such as, but not limited to, a chemotherapeutic drug or a steroid, antibiotic, vitamin, antibody therapy, gene therapy or the like.

The invention will now be further described by reference to the following non-limiting examples

### EXAMPLES

### Example 1: Chaga Ethanol Extraction

Chaga mushrooms were chopped using a coffee grinder into pieces smaller than about 3 mm. One part chopped Chaga was mixed with two parts ethanol. Water was added to the ethanol/Chaga mixture in a volume ratio of 70:30 ethanol/water. The water/ethanol/Chaga mixture was then left to sit at room temperature for a period of three months, at which time the mixture was filtered using a sieve with an aperture size of around 1 mm so that substantially all solid matter was removed. After filtering, the mixture was stored in a glass jar as the ethanol extraction.

### Example 2: Chaga Reduction

Chaga mushrooms were chopped using a coffee grinder into pieces smaller than about 3 mm. About 1.5 cups of the chopped Chaga was placed in a pot containing around 4.8 liters of room temperature water. The water had been reverse osmosis filtered. The water was heated and brought to a strong, rolling boil for 4-5 minutes and then the temperature reduced to maintain a low boil for about 20-30 minutes. The heat was removed, and the water allowed to cool for about thirty minutes, so that the cycle of heating and cooling took about one hour. The cycle was repeated five to six times over a period of 5-6 hours. The Chaga/water mixture was filtered using a sieve with an aperture size of around 1 mm so that substantially all solid matter was removed from the water. After cooling and filtering the mixture was stored as the Chaga reduction.

### Example 3: Extraction/Reduction Mixture

One part by volume of the ethanol extraction was added to five parts by volume of the Chaga reduction in a glass container to form the extraction/reduction mixture. The extraction/reduction mixture is also referred to below as "the oral formula solution."

### Example 4: Distillate

The extraction/reduction mixture was distilled by heating the mixture up to a temperature where steam was first detected to come off the mixture. The matter coming off the mixture was cooled and condensed to form the distillate. The amount of distillate achieved was in the range 30-45 ounces, from an ER mixture of 384 oz. Thus, the remaining fraction was present in an amount of 339-354 ounces.

### Example 5: Oral Formulation I

Oral Formulation I was prepared by mixing the reduction, the extract and the remaining fraction in a weight ratio of 5:1:1.

### Example 6: Oral Formulation II

Oral Formulation **II** was prepared by mixing the reduction, the extract, the remaining fraction, the manuka honey and the coconut oil in a weight ratio of 10:2:2: 1: 1.

### Example 7: Topical Formulation I

Equal portions by volume of remaining fraction and ethanol extraction were mixed together to form an intermediate mixture to which was added manuka honey in a volume ratio of 8 parts intermediate mixture and one part manuka honey. The mixture with honey was stirred to dissolve the honey. Coconut oil was then added to the honey-containing mixture, about 1 part coconut oil to 1 part honey-containing mixture, by volume, to make Topical Formulation I.

### Example 8: Topical Formulation II

Formulation II is prepared using the same procedure as Formulation I, except that the intermediate mixture contained two parts Chaga reduction to one part distillate and one part ethanol extraction. The active ingredient in all formulations described in these Examples is the chemical compound represented in Formula 1.

### Example 9: Case Studies

The Chaga-based formulations and mixtures described above were administered to a number of equine and human subjects suffering from cancers of different types. Each case study below describes the way I which the Chaga-based formulation and mixture was administered and the progress of the cancer following administration.

### Case Study 1: Equine Subject 1 - Metastatic Melanoma

A nine-year-old grey thoroughbred gelding was found to have tumors growing in the region of its esophagus and was subsequently diagnosed with multiple metastatic melanoma tumors encroaching on its esophagus and growing rapidly. The size of the tumors at the time of diagnosis were approximately 5cm x 6cm. Consultation with an expert resulted in a plan for euthanasia before the tumors progressed to the point of impairing all functions of the esophagus. There were no surgical options. The consulting veterinarian advised against performing a biopsy with the risk of aggravating the cancer cells and causing the cells to metastasize at an accelerated rate, knowing the most likely cause of the tumors to be accurate.

The patient was started on a Chaga/manuka regimen with the hope that the regimen was slow the progression of the tumors, giving the patient more time before euthanasia. The regimen contained an oral component and a topical component. The oral component included administering the oral formula solution to the patient once a day for 90 days. The mixture was administered by adding to the patient's feed. The topical component included applying Formulation **I** every 12-24 hours over the tumor site by spraying using a hand pump spray.

After 90 days of treatment the patient's owner confirmed with the consulting veterinarian via visual inspection that the tumors had resolved and there was no visual or physical evidence of the tumors. The patient continued to receive the oral formula and topical formula application for another 60 days. There was no recurrence of the melanoma tumors in a two-year period following the first administration of the Chaga/manuka regimen.

### Case Study 2: Equine Subject 2 - Squamous Cell Carcinoma

A 14 year old spotted draft horse was identified to have an approximately 10 cm x 7.5 cm foul-smelling, non-healing, deep first lesion at the left of the tail head, in addition to a visible second lesion, about 20 mm across, distal to the first region. A third lesion, about 18 mm across, formed of thickened skin, was felt under the skin and was positioned medial and distal to the second lesion. Two areas of the lesion were biopsied for histopathology. The preliminary sections were initially found to contain eosinophilic/granulomatous inflammation. The lesion was later confirmed to contain squamous cell carcinoma (SCC).

On day 0, those parts of the SCC tumor that were deemed safe to remove were surgically excised, with suspected unclean margins. The surgery left an open lesion approximately 7.5 cm x IO cm x 28 cm. The surgeon was concerned about permanent nerve damage to the patient's tail due to the rapid growth of the SCC. On day 3 the patient was started on a Chaga/Manuka regimen. Topical Formulation **I** was applied at the lesion site at least once every 48 hours for 77 days. Oral Formulation **I** was administered daily during the final two weeks of the 77-day period, by sprinkling 10 oz. of the solution on the patient's feed.

There was rapid improvement in the lesion during treatment with the Chaga/Manuka regimen. The lesion had significantly disappeared at 59 days. The patient's primary treating physician confirmed with visual inspection that all SSC, both confirmed and suspected, had been resolved.

Due to the area of concern, close monitoring and clearing of debris continued. The involvement of nematodes at the lesion site required the additional use of skin ointments to prevent recurrence. The patient continued to receive a single 10 oz. dose of Oral Formulation **I** every second day over a 6-week period of time. The patient was on a first course of antibiotics for three weeks, starting at day 134 to treat scalding arising from the nematode infection. The patient started a daily dose of half strength (5 oz./day) on day 165 until around day 203, at which point the dose was returned to 10 oz./day. The patient underwent a second course of antibiotics between days 186 and 207. As of day 223, the original cancer lesion site had remained clear to visual inspection and hair had begun to regrow at the lesion site.

### Case Study 3: Equine Subject 3: Melanoma

A 10-year-old gray Irish Sport Horse gelding was diagnosed with multiple sarcoid and melanoma growths. On day 0, the melanoma was removed from the left neck near the nuchal ligament. A sarcoid growth was removed from the right medial stifle. The patient was given ceftiofur (antibiotic) and flunixin (non-steroidal anti-inflammatory) Following treatment, some masses regressed while others regrew. The sutures were removed on day 21, when tumor regrowth was observed. Sarcoid implantation was made in the left neck. The patient continued to have subcutaneous tumors around the neck area.

Around day 130, The patient began treatment using the Chaga/Manuka regimen. Topical Formulation I was applied to the tumors at the neck area at the neck lesion site at least every 48 hours. Oral Formulation I was administered daily by sprinkling 10 ounces of the solution on the patient's feed. Around day 190, the patient's owner reported that the patient's response to the regimen was positive, and that the tumors located on both sides of his neck and head had receded significantly in size and shape. The remaining tumor mass was reported to have changed in feel, based on palpation. The remaining mass was reported as feeling like a "regular" melanoma mass, without the palpable internal ridges in conjunction with the mobile soft tissue surrounding it commonly associated with a growing melanoma. The patient continued on the Chaga/Manuka regimen until about day 220.

Around day 365 and later the patient returned to competition for the summer and fall seasons. The patient was still alive at the time of writing, around day 585.

### Case Study 4: Human Subject 1: Prostate Cancer

A patient's PSA was measured to be 66.75 on day 0, amid concerns of bone lesions. On day 42, the patient was diagnosed with prostate cancer with a Gleason score of 7, classifying the cancer as medium grade. The patient was initially started on hormone therapy with the hope that this would slow the progression of the prostate cancer. The patient was also notified of concerning bone lesions on his scans. The patient was started on degarelix on day 42. On day 72 the patient was started on bicalutamide. Around day 92 the patient's PSA measured 64. On day 112, the patient was diagnosed as having basal cell carcinoma (BCC) and squamous cell carcinoma (SCC). The BCC and SCC sites were surgically removed around day 122.

The patient began voluntarily using the Chaga/Manuka formula on a daily basis around day 92, by adding about 2 oz. of Oral Formulation I to a cup of coffee. The patient also voluntarily started on other herbal supplements. The patient continued to experience recurrent kidney infections, possibly due to the location and size of the prostate tumor through to around day 145. The patient had also had a catheter placed on day 0 and was still using the catheter through day 145. On day 154 the patient's PSA measured 13.25.

Around day 154 the patient was able to have the catheter removed and has not needed a catheter placed since. Around day 214, the patient's PSA measured 6.4. The patient's PSA remained below 8 until a measurement at around day 440 confirmed a PSA level of 11.9. It turns out the patient had stopped taking the oral formula solution at around day 381. The patient resumed the Chaga/Manuka regimen around day 440.

After day 440, the patient's cancer diagnosis was updated to benign, and he continued to use the Chaga/Manuka regimen. On day 560, the patient's PSA was measured to be 6.25 and his health status was maintained.

### Case Study 5: Human Subject 2: Colorectal Cancer

A male patient around 74 years old was diagnosed with stage IV colorectal cancer that had metastasized to the liver, lungs and kidney. The cancer was considered to be inoperable. After two years of chemotherapy treatment, including experimental treatments, the patient was accepted into hospice care, was receiving palliative care and was given a life expectancy of 2-6 months. One month after entering hospice care the patient started a regimen of Oral Formulation **II.** In days 1-7, the patient was administered 8 oz. of Oral Formulation **II** daily. On day 8 and subsequently, the patient was administered 4 oz. of Oral Formulation **II** daily. The patient's "cancer cough" subsided throughout the first two weeks of treatment and was gone by day 15. The patient reported improved appetite, reduced pain, improved sleep cycles and improved eyesight by around day 45. Around day 55 the patient started walking two miles daily. Around day 105 it was confirmed with lab test results the patient's CEA marker had dropped to 9.1 from the starting measurement on Day 0 of 875.

### CASE STUDY 6: Human Patient with Breast cancer metastasized to Leptomeningeal Carcinomatosis

LMC is a 100% lethal diagnosis, with survivability without chemotherapeutic treatment less than 8 weeks and with treatment 2-3 months, up to 12 months in some cases. Six weeks post-diagnosis of LMC, a patient, who had opted out of chemotherapeutic care due to no long-term survivability rate, began a daily oral dose of 5-32oz of Oral Formulation II. From Day 0-120 the patient maintained normal functions of living. Test results around Day 125 showed normal standard CBC markers and normal CEAmarkers. Patient has remained stable with daily functions of living past Day 300.

### Example 10: In Vitro Cell Viability and ATP/ADP Ratio Studies

**Materials:** The cells used in the study-SK Mel 28, MDA-MB-231, and HepG2 were originally obtained from ATCC (American Type Culture Collection, Manassas, VA) and maintained per the following protocols described below. The five samples tested in this study were prepared. Samples 1-4 are various components of the final formulation represented in Formula 1. Therefore, this Example is a comparison of the anti-cancer activity of these various components used in the production of the chemical compound of Formula 1 versus the chemical compound of Formula 1 itself in order to show the increased bioavailability and functions of the chemical compound of Formula.

Sample 1 is the reduction component used to produce the medicinal composition; Sample 2 is the extraction component used to produce the medicinal composition; Sample 3 is the distillate only created during the production of the medicinal composition; Sample 4 is a mixture of the reduction, extraction, distillate, and the chemical compound represented by Formula 1. Samples 5 and 6 are different concentrations of the chemical compound represented by Formula 1 with Sample 6 being a higher concentration of Formula 1 than Sample 5. The materials ELDT-100 (Lot: CC01A25) and CQBL-05K (Lot: CB08A31) were supplied by BioAssay Systems (Hayward, CA).

**Method:** The SK-MEL-28 and HepG2 cell lines from ATCC were grown in T75 culture flasks with ATCC recommended media supplemented with antibiotics (10v% FBS, 1w% streptomycin/penicillin, Eagles Minimum Essential Medium) and grown in incubator at 5% CO2 and 37°C. The MDA-MB-231 cell line was obtained from ATCC and grown in T75 culture flasks using complete media as described in Huang Z et al. (Onco Targets Ther. 2020; 13:5395-5405) and supplemented with antibiotics (10v% FBS, 1w% streptomycin/ penicillin, Roswell Park Memorial Institute 1640 medium) and grown in incubator at 5% CO2 and 37°C. Cell lines were grown to 70-80% confluency prior to seeding in 96-well plates for ELDT-100 and CQBL- 05K.

For the EnzyLight^{™} ATP Assay (EATP-100, BioAssay, Hayward, CA), the SK Mel 28 and MDA- MB-231 cell lines were seeded at a density of 5,000 cells per well while the HEPG2 cells were seeded at a density of 2,500 cells per well in a 96- well white opaque tissue culture plate then treated with the various Samples at 1x, 0.1x, 0.01x, and 0.001x dilutions for 48hrs. Compound dilutions were made in diH2O. Media was removed after 48hrs and then ATP Reagent was added and read on luminometer after one (1) minute. Blanks were obtained by treating and measuring cell-free complete media, and cells not treated with one of the Samples served as the control.

For the CellQuanti-Blue^{™} Cell Viability Assay (CQBL-05K, BioAssay, Hayward, CA), cell lines were seeded at a density of between 16,000 to 20,000 cells per well in a 96-well clear bottom black tissue culture plate. (Each experiment was plated at a uniform density of cells.) Cells were then treated with one of the Samples at various dilutions and incubated for 48hrs. Compound dilutions were made in diH2O. The CellQuanti-Blue Reagent was then added to wells and incubated for 1hr at 37°C. Fluorescence was then measured at 530nm excitation and 590nm emission. Blanks were obtained via cell-free complete media and by treating cells with reconstituted 1w% saponin.

Standard error was calculated using Propagation of Error methodology (see www.chem.libretexts.org/Bookshelves/Analytical_Chemistry/Supplemental_Modules_(Analy tical_Chemistry)/Quantifying_Nature/Significant_Digits/Propagation_of_Error.). Samples were read on a Molecular Devices SpectraMax M2 instrument (BioAssay, Hayward, CA).

**Results:** The results of the ATP/ADP ration studies and cell viability studies for each cell line and each of the five Samples, including the compound represented by Formula 1, are shown in the Figures as indicated below.

**SK-Mel 28** The SK-Mel 28 cell line demonstrated significant inhibition of ATP levels with Samples 1, 2, 4, and 5 at 1X concentration with restoration of ATP levels as the sample levels decreased (Fig. 1). Fig. 1 provides relative ATP levels of SK Mel 28 cells following treatment with compounds.

SK Mel 28 cells were plated as described in the Method then grown for 24 hours before the relative ATP and ADP levels were measured. The relative luminescence (RLU A measurement) of SK Mel 28 cells represents the relative ATP levels present in the cells.

Sample 3 showed no effect in the SK-Mel 28 cell line. Unfortunately, the ADP portion of the ATP/ADP ratio assay was less effective, so a clear picture of apoptosis versus necrosis (necroptosis) was not as clear (Fig. 2). Figure 2 provides Relative ADP levels of SK Mel 28 cells following treatment with compounds.

SK Mel 28 cells were plated as described in the Methods then grown for 24 hours before the relative ATP and ADP levels were measured. The relative luminescence (RLU C - RLU B measurement) of SK Mel 28 cells represents the relative ADP levels present in the cells following compound treatment.

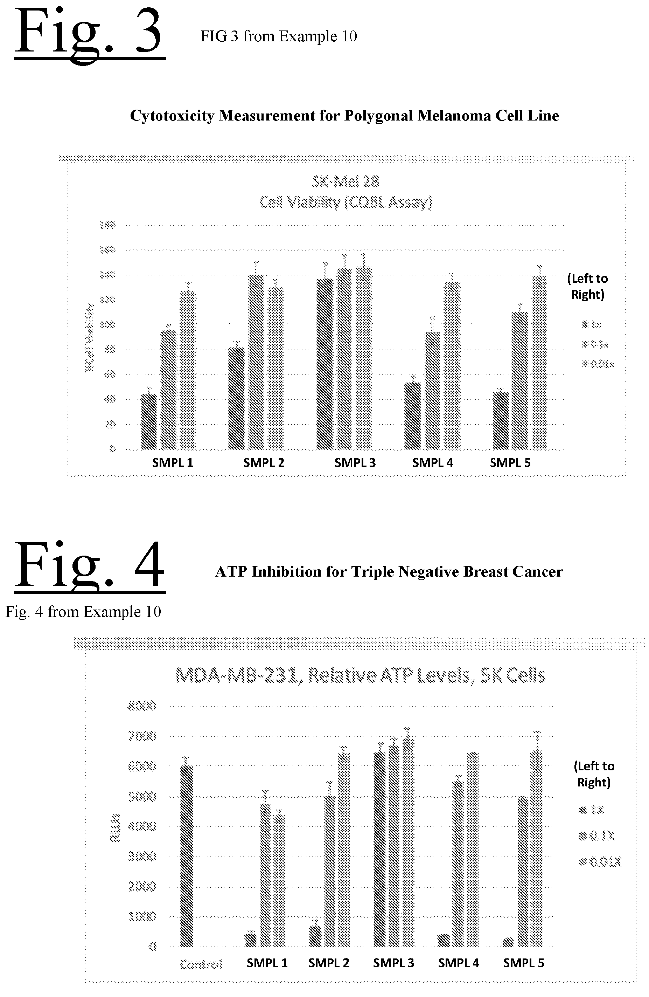

The Cell Viability assessment showed a similar trend to the ATP levels, but with a clearer gradation (Fig. 3). For example, Compound 5 at 0.1X and 0.01 X had relative ATP values within the same range, but the Cell Viability Assay showed a clear distinction at all three concentrations. Fig. 3 provides a cell viability assessment of SK-Mel 28 cells using the CellQuanti-Blue^{™} Cell Viability Assay showing the percent viability versus with respect to untreated cells.

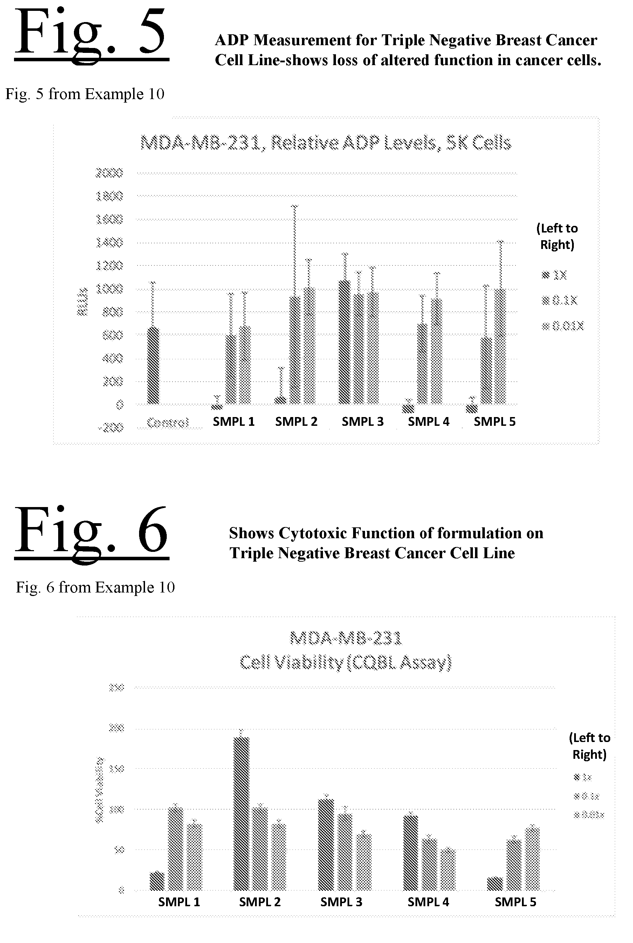

**MDA-MB-231** The MDA-MB-231 cell line demonstrated very significant inhibition of ATP levels with Samples 1, 2, 4, and 5 at 1X concentration with restoration of ATP levels as the sample levels decreased (Fig. 4). Figure 4 provides relative ATP levels of MDA-MB-231 cells following treatment with the samples. MDA-MB-231 cells were plated as described in the Methods then grown for 24 hours before the relative ATP and ADP levels were measured. The relative luminescence (RLU A measurement) of MDA-MB- 231 cells represents the relative ATP levels present in the cells. Sample 3 showed no effect in the MDA-MB-231 cell line. The ADP portion of the ATP/ADP ratio assay was more effective than the SK-Mel 28, but a clear picture of apoptosis versus necrosis (necroptosis) could not be obtained at 24 hours (Fig. 5). Fig. 5 provides relative ADP levels of MDA-MB-231 cells following treatment with the samples. MDA-MB-231 cells were plated as described in the Method then grown for 24 hours before the relative ATP and ADP levels were measured. The relative luminescence (RLU C - RLU B measurement) of MDA-MB-231 cells represents the relative ADP levels present in the cells following compound treatment.

It was suspected that at 1X sample we are seeing necrosis (necroptosis), but at the lower concentrations, we are starting to see apoptosis. The Cell Viability assessment showed an unusual trend for Samples 2, 3, and 4 with an inverse relationship in evidence (Fig. 6). Fig. 6 provides a cell viability assessment of MDA-MB-231 cells using the CellQuanti-Blue Cell Viability Assay. The percent viability with respect to untreated cells is shown. For Sample 5, the normal relationship was in evidence with cell viability increasing with decreasing sample concentration. The behavior of Sample 1 was at odds with the other four samples, showing no clear trend in cell viability.

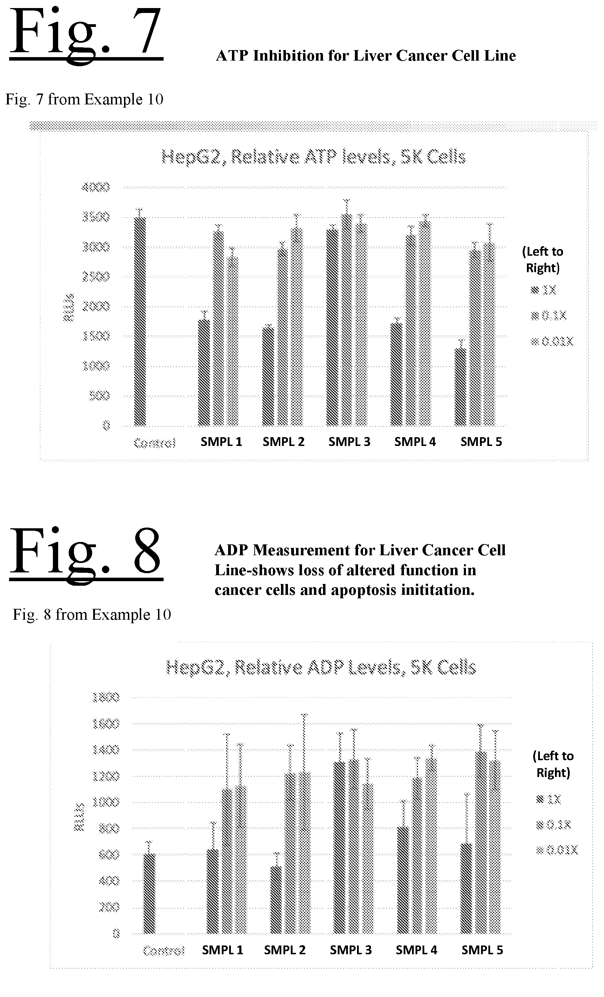

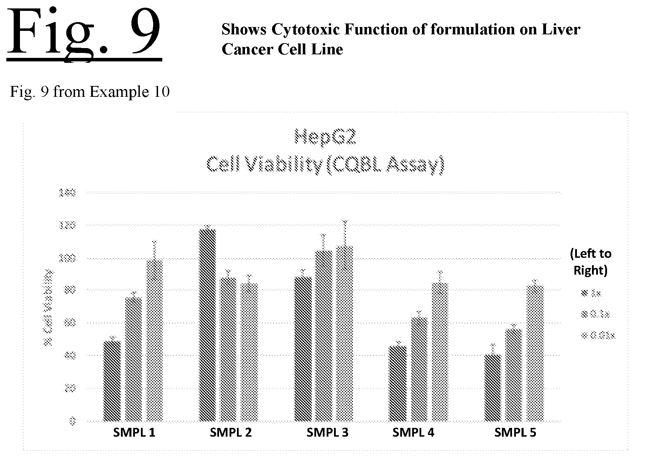

**HepG2** The HepG2 cell line demonstrated significant inhibition of ATP levels with Compounds 1, 2, 4, and 5 at 1X concentration with restoration of ATP levels as the sample levels decreased (Fig. 7). Fig. 7 provides relative ATP levels of HepG2 cells folling treatment with the samples. HepG2 cells were plated as described in the Methods then grown for 24 hours before the relative ATP and ADP levels were measured. The relative luminescence (RLU A measurement) of HepG2 cells represents the relative ATP levels present in the cells. Sample 3 showed no effect in the HepG2 cell line. The ADP portion of the ATP/ADP ratio assay was more effective with the HepG2 cell line (Fig. 8). Fig. 8 provides relative ADP levels of HepG2 cells following treatment with the samples. HepG2 cells were plated as described in the Methods then grown for 24 hours before the relative ATP and ADP levels were measured. The relative luminescence (RLU C - RLU B measurement) of HepG2 cells represents the relative ADP levels present in the cells following sample treatment. There are indications of apoptosis at 0.1X and 0.01X concentrations and probable necrosis (necroptosis) at 1X concentrations. The Cell Viability assessment showed a similar trend to the ATP levels with Samples 1, 4, and 5, but again Sample 2 showed an inverse relationship with cell viability and sample, with decreasing sample leading to decreased cell viability (Fig. 9). Fig. 9 provises a cell viability assessment of HepG2 cells using the CellQuanti-Blue Cell Viability Assay. The percent viability with respect to untreated cells is shown.

**Conclusions:** All three cell lines (SK-Mel 28, MDA-MB-231, and HepG2) showed similar behavior with all five samples in the ATP Assay. A 1X dilution of Samples 1, 2, 4, and 5 inhibited ATP production with restoration of ATP production with increasing dilution of sample. The ADP component was much less clear with apoptosis vs. necrosis (necroptosis) between the three cell lines with only HepG2 giving reasonable performance. Interpretation of the Cell Viability shows inverse behavior observed with Samples 2, 3, and 4 with the MDA-MB-231 cell line.

### Example 11: In Vitro Cell Viability and ATP Studies

**Materials:** The cells used in the study-SK Mel 28, MDA-MB-231, and HepG2 were originally obtained from ATCC (American Type Culture Collection, Manassas, VA) and maintained per the following protocols described below. The samples tested in this study were prepared and stored at 4°C until use. Samples 5 and 6 are different concentrations of the chemical compound represented by Formula 1 with Sample 6 having a 10% higher concentration of the chemical compound represented by Formula 1 than Sample 5. The EATP-100 (Lot: CC11A01) and CQBL-05K (Lot: CC10A03) materials were supplied by BioAssay Systems (Hayward, CA).

**Method:** The SK-MEL-28 and HepG2 cell lines obtained from ATCC were grown in T75 culture flasks with ATCC recommended media supplemented with antibiotics (10v% FBS, 1w% streptomycin/penicillin, Eagles Minimum Essential Medium) and grown in incubator at 5% CO2 and 37°C. The MDA-MB-231 cell line from ATCC was grown in T75 culture flasks using complete media as described in Huang Z et al. (Onco Targets Ther. 2020; 13:5395-5405) and supplemented with antibiotics (10v% FBS, 1w% streptomycin/ penicillin, Roswell Park Memorial Institute 1640 medium) and grown in incubator at 5% CO2 and 37°C. Cell lines were grown to 70-80% confluency prior to seeding in 96-well plates for ELDT-100 and CQBL- 05K.

For the EnzyLight^{™} ATP Assay (EATP-100, BioAssay, Hayward, CA), the SK Mel 28 and MDA-MB-231 cell lines were seeded at a density of 5,000 cells per well while the HepG2 cells were seeded at a density of 2,500 cells per well in a 96- well white opaque tissue culture plate then treated with samples at 1x, 0.1x, 0.01x, and 0.001x dilutions for 48hrs. Compound dilutions were made in diH2O. Media was removed after 48 hrs. and then ATP Reagent was added and read on luminometer after one (1) minute. Blanks were obtained by treating and measuring cell-free complete media, and cells not treated with Sample served as the Control. For the CellQuanti-Blue^{™} Cell Viability Assay (CQBL-05K, BioAssay, Hayward, CA), cell lines were seeded at a density of between 16,000 to 20,000 cells per well in a 96-well clear bottom black tissue culture plate. (Each experiment was plated at a uniform density of cells.) Cells were then treated with samples at various dilutions and incubated for 48hrs. Compound dilutions were made in diH2O. The CellQuanti-Blue Reagent was then added to wells and incubated for 1hr at 37°C. Fluorescence was then measured at 530nm excitation and 590nm emission. Blanks were obtained via cell-free complete media and by treating cells with reconstituted 1w% saponin. Standard error was calculated using Propagation of Error methodology (see www.chem.libretexts.orgBookshelves/
Analytical_Chemistry/Supplemental_Modules_(Analytical_Chemistry)/Quantifying_Nature/ Significant_Digits/Propagation_of_Error.) Samples were read on a Molecular Devices SpectraMax M2 instrument (BioAssay, Hayward, CA).

**Results:** The results of the ATP studies and cell viability studies for each cell line and each of the five samples are shown in the Figures.

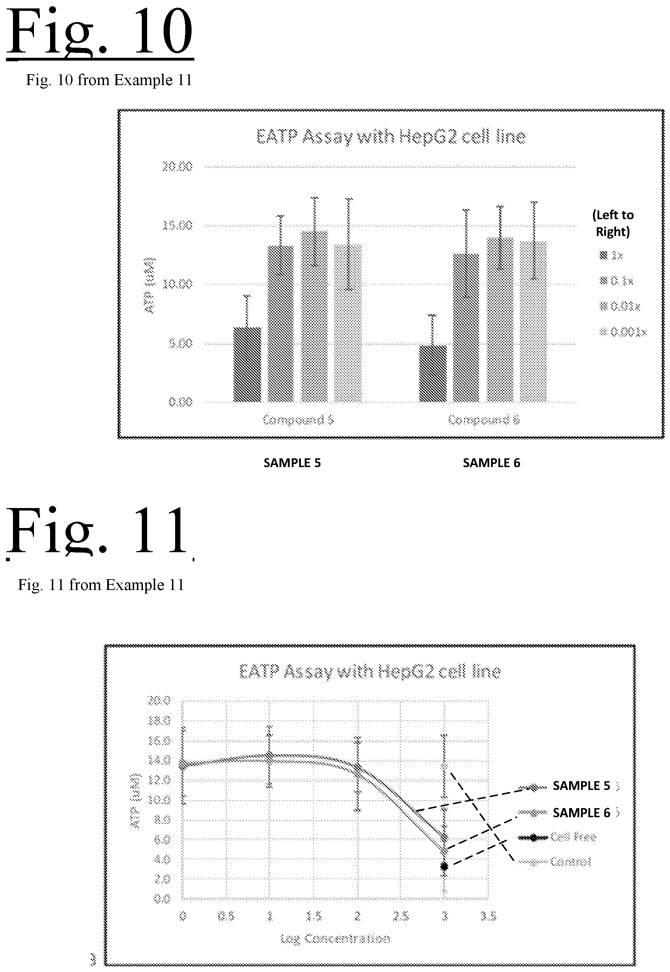

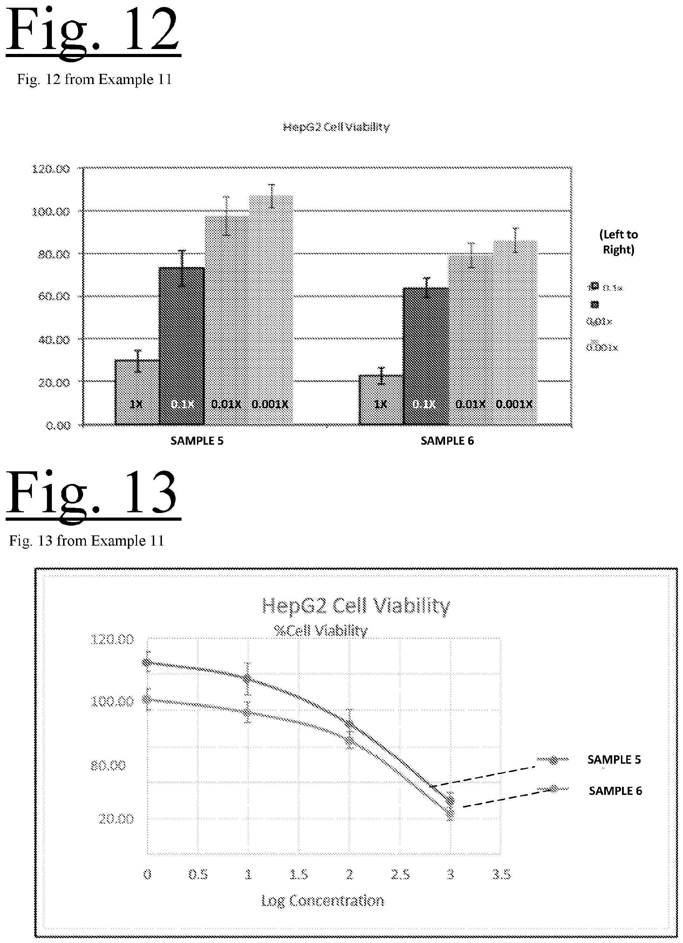

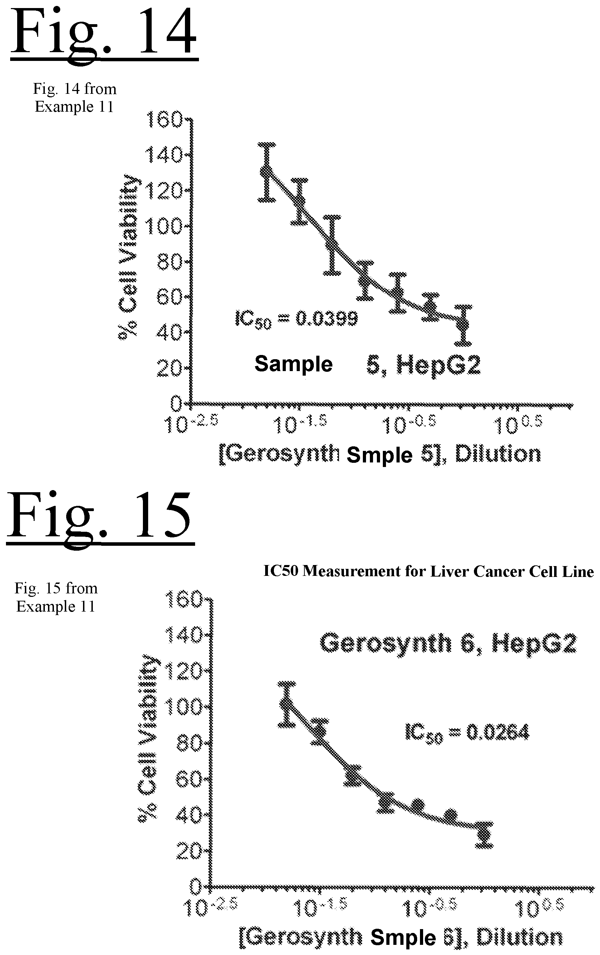

**HepG2** The HepG2 cell line demonstrated inhibition of ATP levels with Samples 5 and 6 at 1X concentration with restoration of ATP levels as the sample levels decreased (Fig. 10). The data was log transformed to better visualize the data (Fig. 11). The Cell Viability assessment showed a similar trend to the ATP levels with both samples (Figs. 12 and 13). With the Cell Viability measurement, Sample 6 demonstrated greater cytotoxicity compared to Sample 5, especially at lower dosages. Relative IC50 values were obtained for both Sample 5 and Sample 6 with Sample 6 being the more potent in relative terms (Figs. 14 and 15).

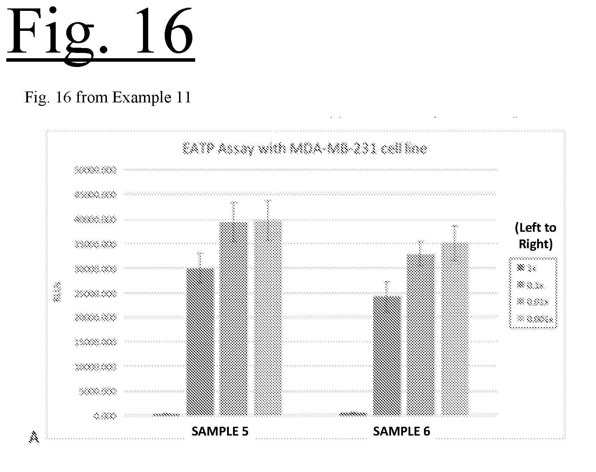

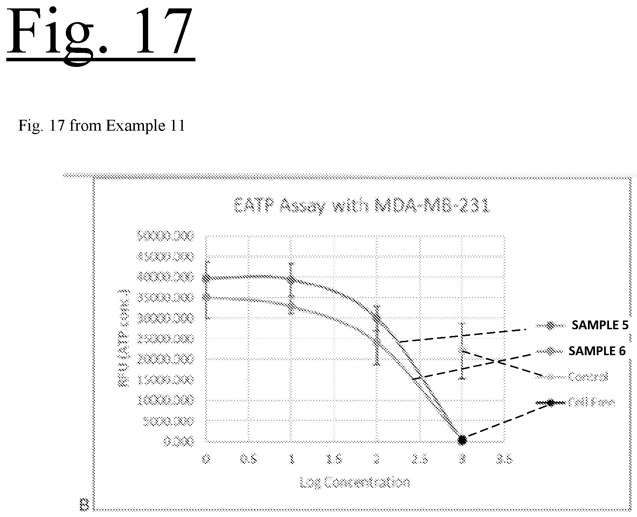

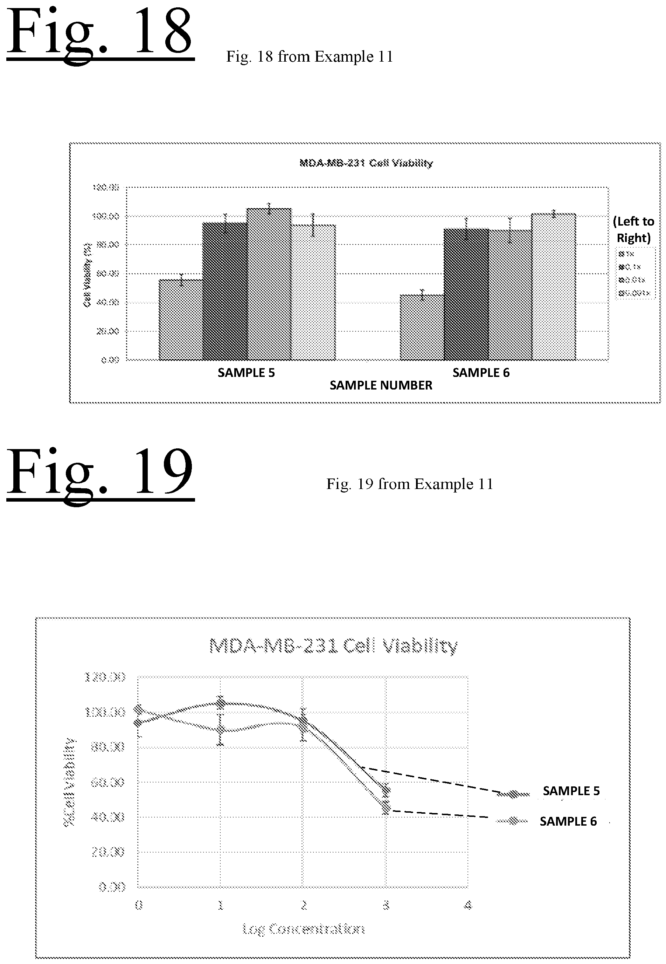

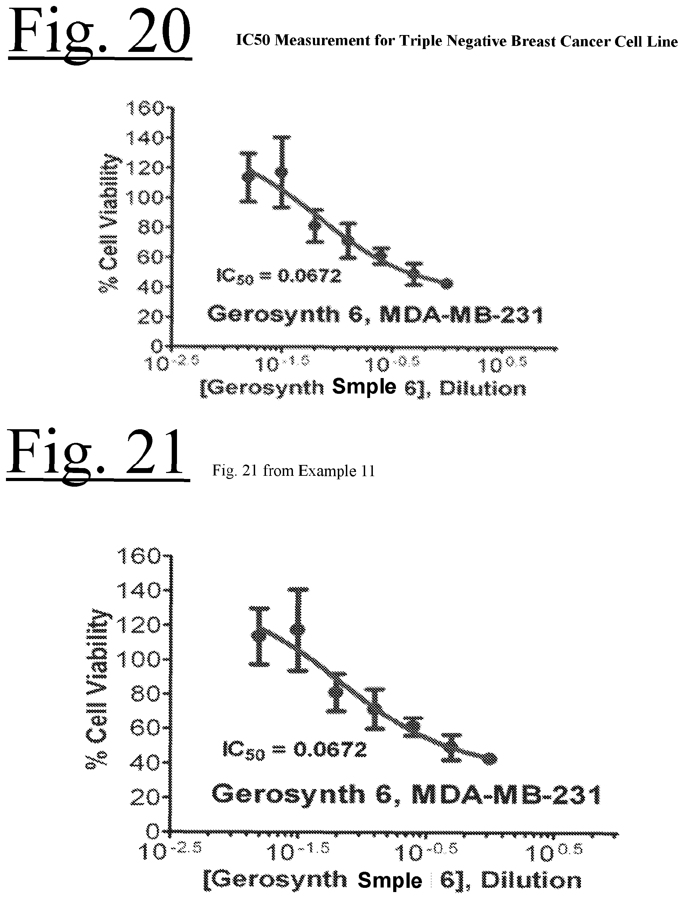

**MDA-MB-231** The MDA-MB-231 cell line demonstrated significant inhibition of ATP levels with Samples 5 and 6 at 1X concentration with restoration of ATP levels as the sample levels decreased (Fig. 16 and 17). For reasons it was not possible to ascertain, ATP quantification proved challenging (ATP standard curves were the issue), so only the relative ATP levels are shown. For both Samples 5 and 6, the expected relationship was in evidence with cell viability increasing with decreasing sample concentration (Figs. 18 and 19). The IC50 determination showed that Sample 6 had greater cytotoxicity in MDA-MB-231 cells as opposed to Sample 5 (Figs. 20 and 21).

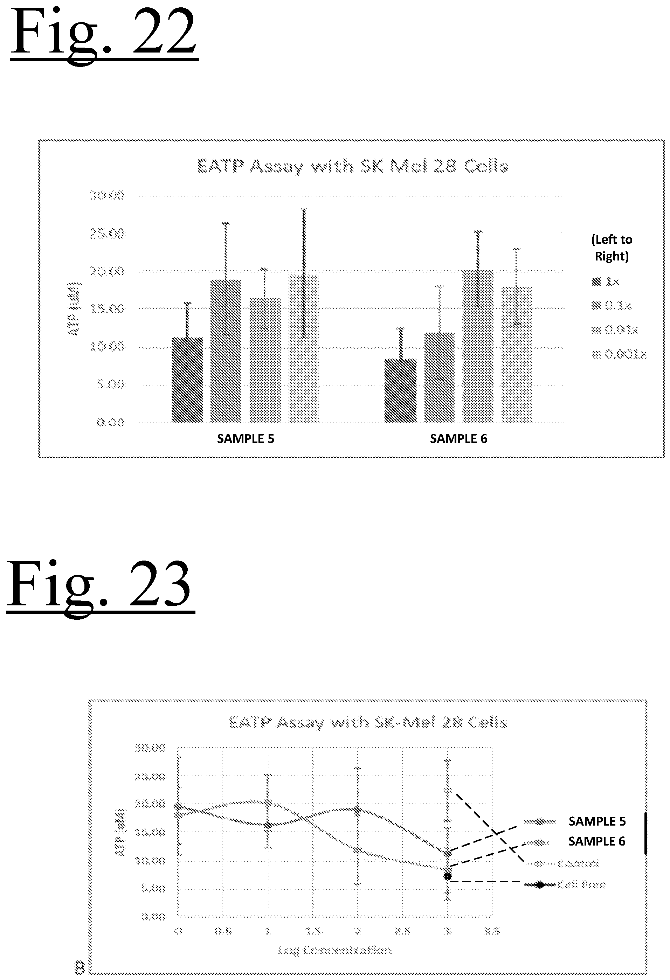

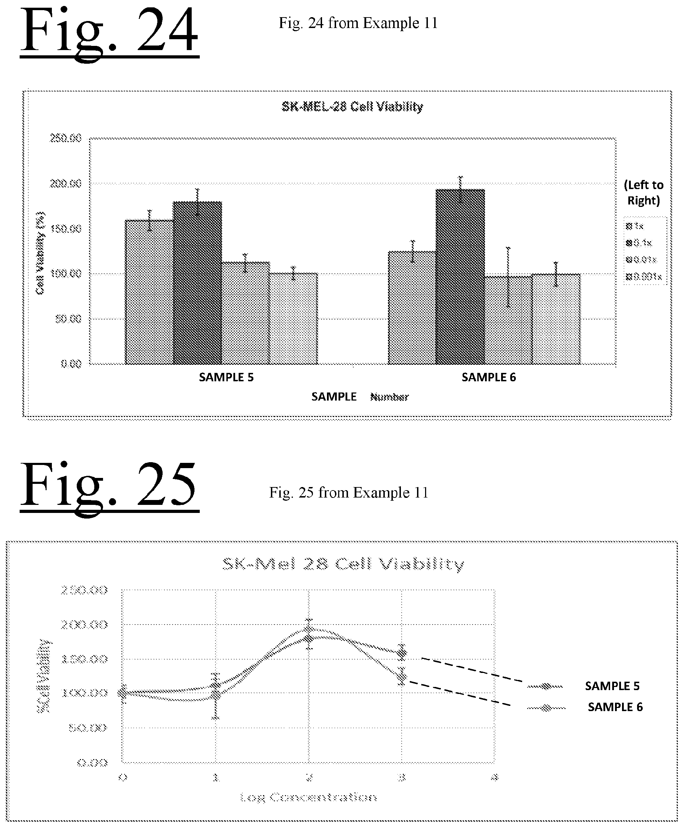

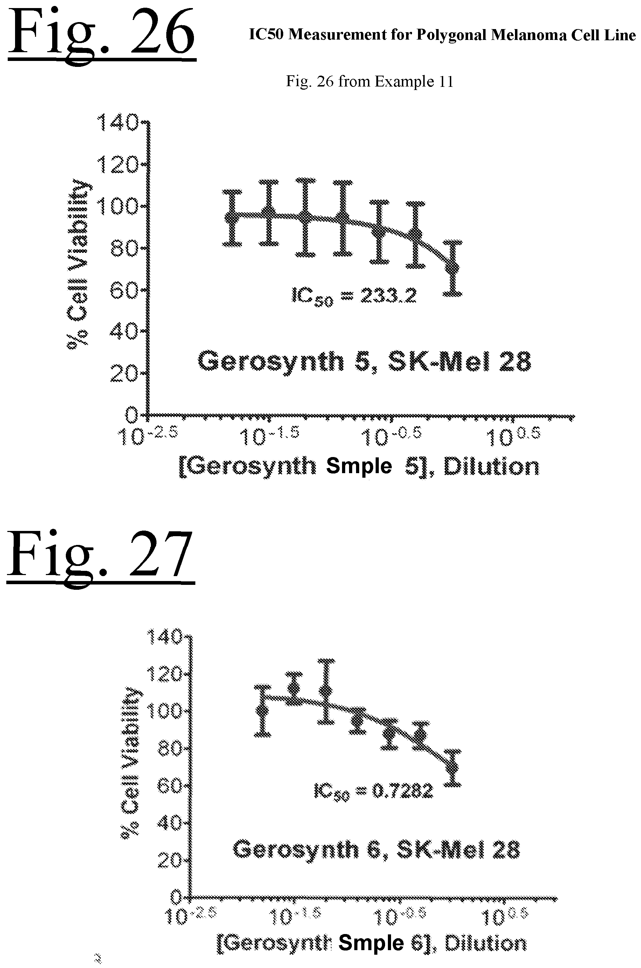

**SK-Mel 28** The SK-Mel 28 cell line demonstrated inhibition of ATP levels with Compounds 5 and 6 at 1X concentration with restoration of ATP levels as the sample levels decreased (Figs. 22 and 23). As in the previous studies, the ATP picture is less clear than with HepG2 and MDA-MB-231. (Figs. 24 and 25). The Cell Viability assessment was less clear, which is contrary to the previous work showing a clear impact to Cell Viability with Sample 5. (Figs. 26 and 27). The IC50 assessment demonstrated that the samples were less toxic to the SK- Mel 28 cells as compared to MDA-MB-231.

**Conclusions:** Samples 5 and 6 demonstrated similar behavior in MDA-MB-231 and HepG2 cells lines, which was consistent with the results obtained in the study described in Example 10 above. The behavior of the SK-Mel 28 cell line was more erratic and did not replicate the previous results with the exception of the ATP assay, which did show some similarities to the previous work. This is most likely attributed to the decomposition timeline of the samples. The SK-Mel studies were performed last. In all cases, Sample 6 was more potent than Sample 5 as demonstrated by the IC50 curves. (It should be noted that the IC50 values obtained are relative values as compared to the concentrated 1x Samples.) One of the challenges that complicated the work was synchronizing the cell lines with sample availability as the samples were somewhat labile in nature. There were also difficulties with the HepG2 cell line that necessitated the reacquisition of fresh cells from ATCC.

### Example 12: In Vitro Cell Viability and ATP Studies - Pharmaceutical Studies

**Materials:** The cell lines used in this study-HCT-116, SK Mel 28, MDA-MB-231, and HepG2-were obtained from ATCC (American Type Culture Collection, Manassas, VA) and maintained per the protocols described below. The Samples for this study were prepared and stored at 4°C until use. Samples 7 and 8 are different concentrations of the chemical compound represented by Formula 2 with Sample 7 having a 10% higher concentration of the chemical compound represented by Formula 1 than Sample 8. The EATP-100 (Lot: CD07A13) and CQBL-10K (Lot: CC10A03) materials were supplied by BioAssay Systems (Hayward, CA).

**Method:** The SK Mel 28 and HepG2 cell lines were obtained from ATCC and grown in T75 culture flasks with ATCC recommended media supplemented with antibiotics (10v% FBS, 1w% streptomycin/penicillin, Eagles Minimum Essential Medium) and grown in incubator at 5% CO2 and 37°C. The MDA-MB-231 cell line was obtained from ATCC and grown in T75 culture flasks using complete media as described in Huang Z et al. (Onco Targets Ther. 2020;13:5395-5405) and supplemented with antibiotics (10v% FBS, 1w% streptomycin/ penicillin, Roswell Park Memorial Institute 1640 medium) and grown in incubator at 5% CO2 and 37°C. The HCT-116 cell line was obtained from ATCC and grown in T75 culture flasks with ATCC recommended media supplemented with antibiotics (10v% FBS, 1w% streptomycin/penicillin, McCoy's 5a Medium Modified) and grown in incubator at 5% CO2 and 37°C. Cell lines were grown to 70-80% confluency prior to seeding in 96- well plates for the EATP-100 and CQBL-10K assays.

The cells were treated with Samples 7 and 8, separately, at the following concentrations for 48 hours (Compound dilutions were made in diH2O):
Sample 7: 1372.2, 457.4, 152.5, 50.8, 16.9, 5.65, and 1.88 µM
Sample 8: 1571.8, 523.9, 174.6, 58.2, 19.4, 6.47, and 2.16 µM

For the EnzyLight^{™} ATP Assay (EATP-100, BioAssay, Hayward, CA), the SK Mel 28 and MDA- MB-231 cell lines were seeded at a density of 5,000 cells per well while the HepG2 and HCT-116 cell lines were seeded at a density of 2,500 cells per well in a 96-well white, opaque tissue-culture plate.

Media was removed after 48 hrs. and then ATP Reagent was added and read on luminometer after one (1) minute. Blanks were obtained by measuring cell-free complete media and cells not treated with either sample served as the Control.

For the CellQuanti-Blue^{™} Cell Viability Assay (CQBL-10K, BioAssay, Hayward, CA), the MDA-MB- 231, HCT-116, and HepG2 cell lines were seeded at a density of 10,000 cells per well while the SK Mel 28 cell line was seeded at a density of 20,000 cells per well in a 96-well clear bottom black tissue-culture plate. Cells were then treated with samples at various dilutions indicated above and incubated for 48hrs. The CellQuanti-Blue Reagent was added directly to the wells and incubated for 1hr at 37°C. Fluorescence was measured at 530nm excitation and 590nm emission. Blanks were obtained via cell-free complete media and by treating cells with reconstituted 1w% saponin.

The data was analyzed using Microsoft Excel. The data was log-transformed for plotting in GraphPad Prism 4.0 (BioAssay, Hayward, CA). Standard error was calculated using Propagation of Error methodology in accordance with www.chem.libretexts.org/Bookshelves /Analytical Chemistry/Supplemental Modules (Analytical Chemistry)/ Quantifying_Nature/Significant_Digits/Propagation_of_Error).

Samples were read on a Molecular Devices SpectraMax M2 instrument (BioAssay, Hayward, CA).

**Results:** The summary of IC50 results of the Cell Viability and ATP studies is shown in the table below

| | Cmpd #7 ATP | Cmpd #8 ATP | Cmpd #7 Cell Viability | Cmpd #8 Cell Viability |
|---|---|---|---|---|
| HCT-116 | 23.4 µM | 30.1 µM | 103 µM | 88.5 µM |
| HepG2 | 1.2 µM | 1.4 µM | 89.0 µM | 53.8 µM |
| MDA-MB-231 | 74 µM | 79.6 µM | 131 µM | 125 µM |
| SK Mel 28 | 24.3 µM | 32.4 µM | 15.9 µM | 12.7 µM |

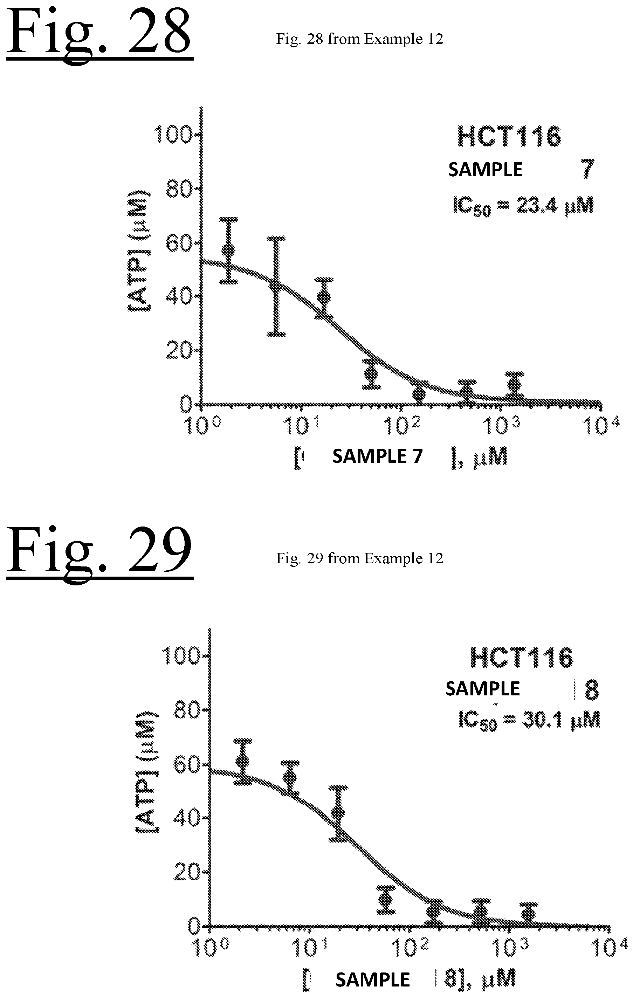

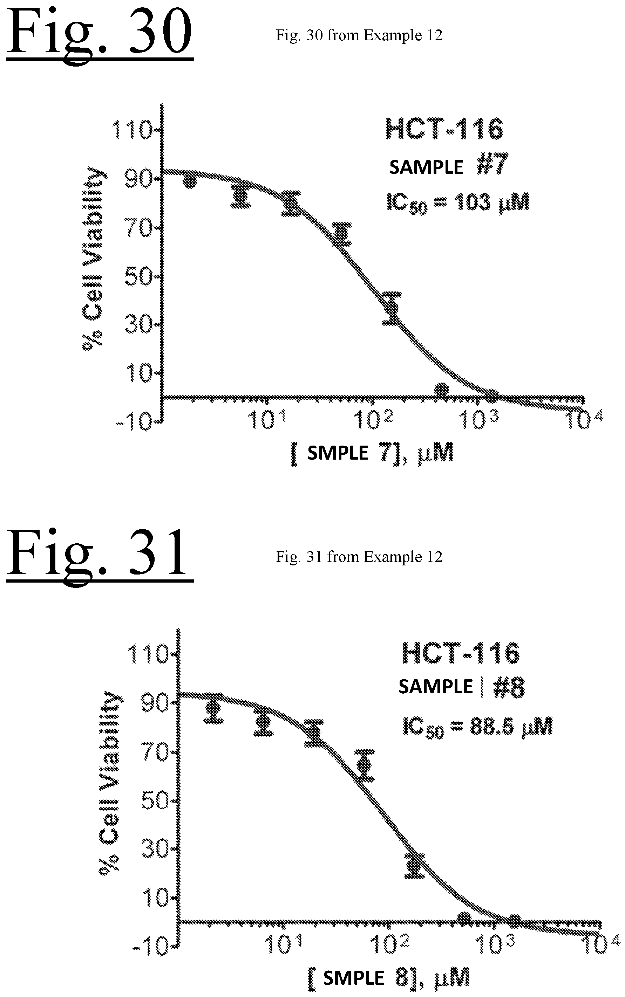

**HCT-116** The HCT-116 cell line demonstrated inhibition of ATP levels with both Samples 7 and 8 (Figs. 28 and 29). The Cell Viability assessment showed an opposite trend to the ATP levels (Figs. 30 and 31). With the ATP measurements, Sample 7 showed evidence of a lower IC50 value as compared to Sample 8. In contrast, from the Cell Viability measurement, Sample 8 demonstrated greater cytotoxicity compared to Sample 7 with a lower IC50 value.

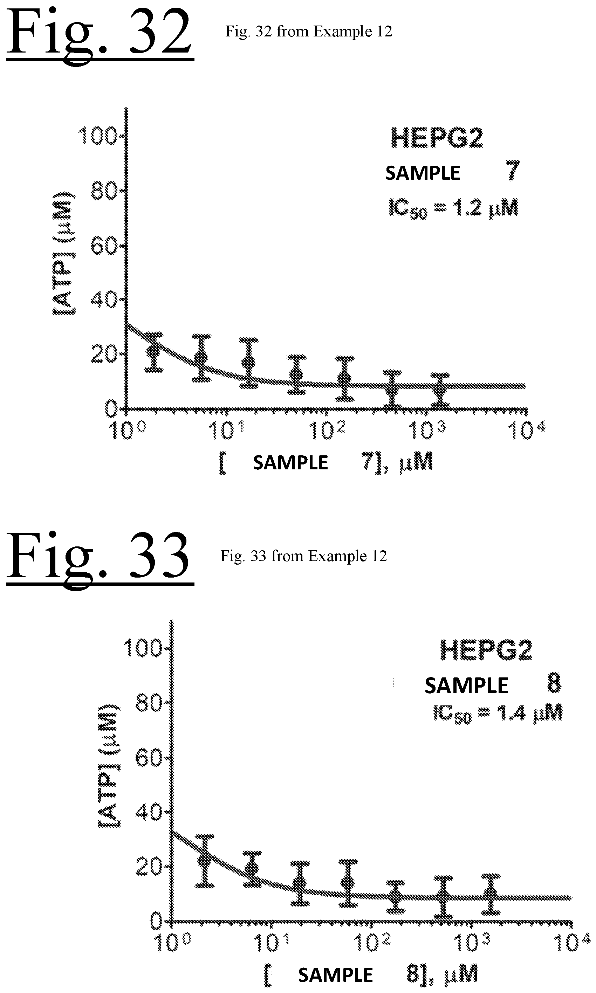

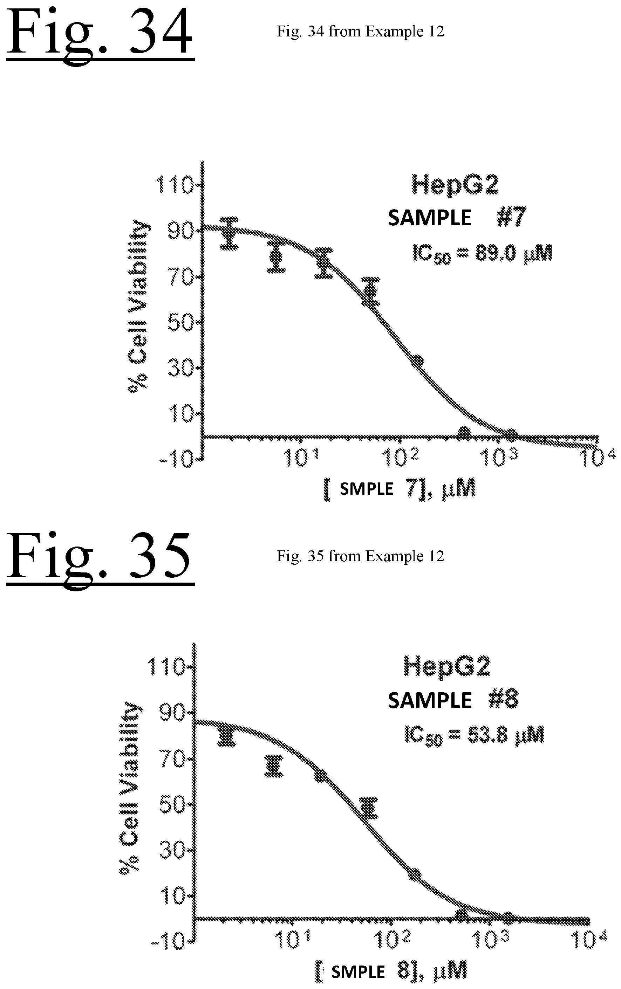

**HepG2** The HepG2 cell line demonstrated inhibition of ATP levels with Compounds 7 and 8 (Figure 32 and 33). The Cell Viability assessment showed a similar trend to the ATP levels with both samples (Figs. 34 and 35). With the Cell Viability measurement, Sample 8 demonstrated greater cytotoxicity compared to Sample 7. IC50 values were obtained for both Samples 7 and 8 with Sample 8 being the more potent.

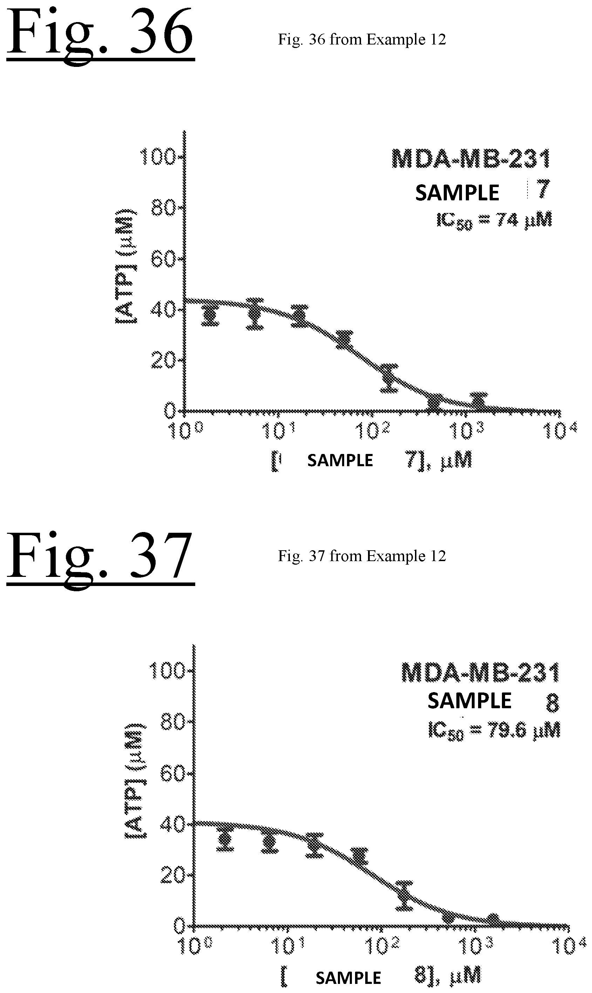

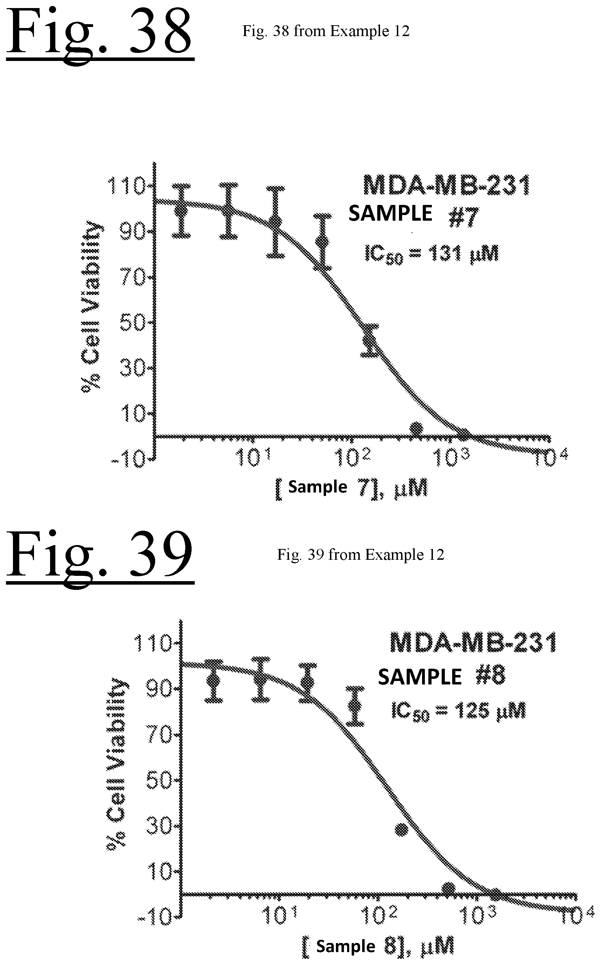

**MDA-MB-231** The MDA-MB-231 cell line demonstrated significant inhibition of ATP levels with Samples 7 and 8 (Figure 36 and 37). The IC50 determination showed that Sample 8 had greater cytotoxicity in MDA-MB-231 cells as opposed to Sample 7 (Figs. 38 and 39).

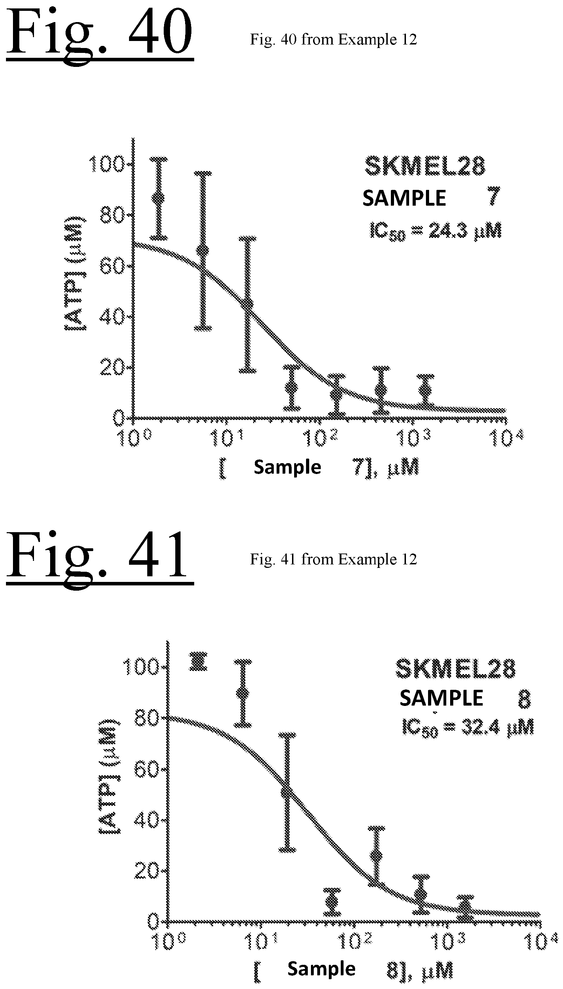

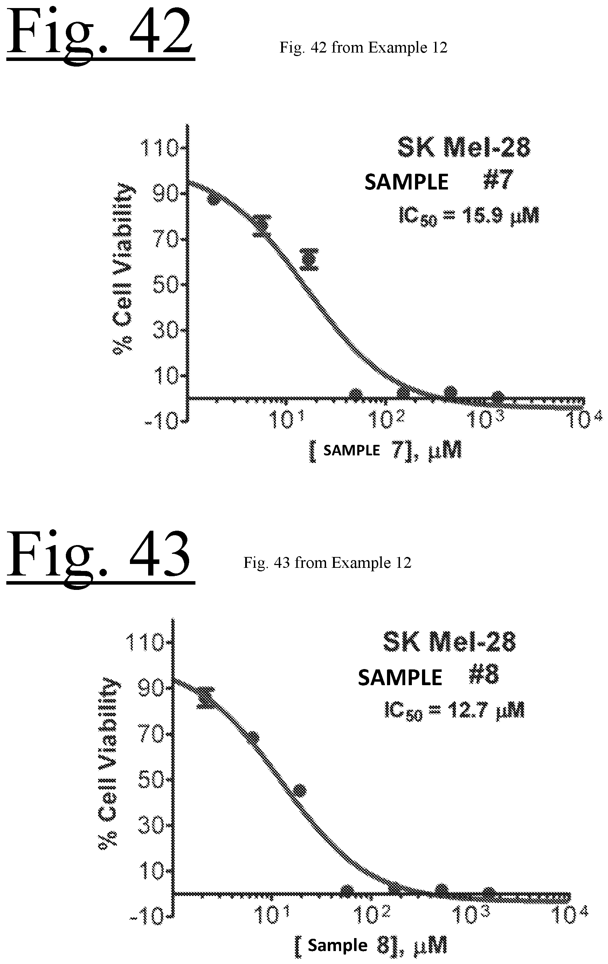

**SK-Mel 28** The SK Mel 28 cell line demonstrated inhibition of ATP levels with Samples 7 and 8 (Figs. 40 and 41). The IC50 assessment of cell viability demonstrated that both samples were more toxic to the SK-Mel 28 cells as compared to the other three cell lines. (Figs. 42 and 43).

The two Samples, 7 and 8, studied in this work demonstrated similar behavior in all four cell lines. The ATP levels were lower when treating with Sample 7, but Sample 8 was more potent than Sample 7 based on cytotoxicity as demonstrated by the IC50 curves using the CQBL-05K assay. It is noteworthy that the ATP levels in HepG2 were significantly lower than the other three cell lines even at the lowest levels of sample treatment. Also noteworthy is the significantly lower IC50 value observed for the cell viability determination with SK Mel 28 as compared to the other three cell lines.

### Example 13: NMR Observations

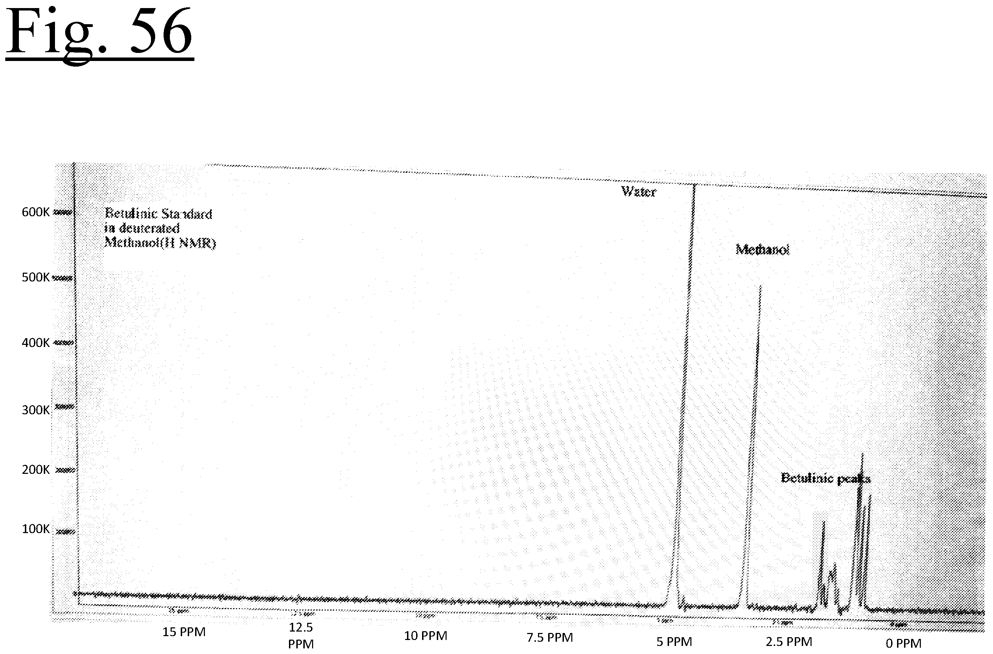

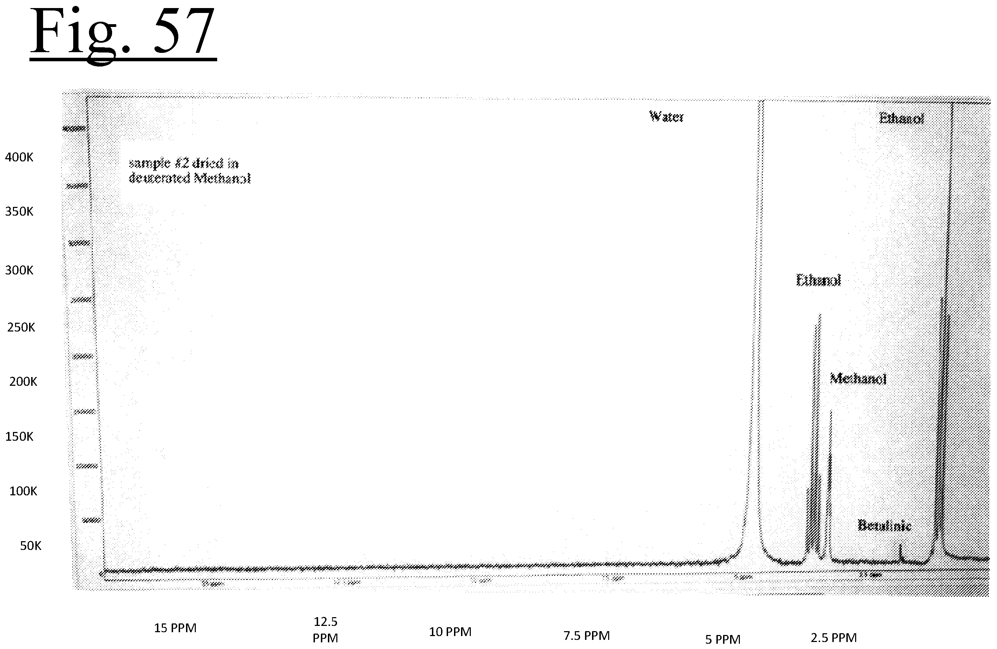

The chemical structure of the compound represented by Formula 1 is confirmed by NMR analysis as shown in Fig. 56 and 57. The percent yield was consistent with what is estimated in public literature for extractions from raw materials post-reaction, and samples analyzed show consistent measurement of percent yield, which improves upon previous research attempts.

The quantity of compound produced by the reaction is sufficient for the proposed activity of the compound, and when isolated and concentrated correctly, leads to a higher percent yield of compound sufficient for clinical use.

Major H peak was chosen for quantification as that is the peak visible in the samples. The azeotropic nature of the water/ethanol extract explains why air drying doesn't allow the solids to progress to complete dryness but does serve well enough to concentrate the sample to more clearly identify the peak. Deuterated methanol was used as the solvent. Secondary betulinic acid peaks found in reference sample are hidden by one of the groupings of peaks for ethanol. Most importantly, the primary betulinic acid peak is easily seen and has been quantified.

Various modifications, improvements and equivalents will be readily apparent to those of skill in the art to which the present invention is directed upon review of the present specification. The claims are intended to cover such modifications, improvements and equivalents. Those skilled in the art will recognize, or be able to ascertain using no more than routine experimentation, many equivalents to the specific embodiments of the invention described herein. Such equivalents are intended to be encompassed by the following claims.

### Example 14: Production of the Chemical Compound Represented by Formula 1

The chemical compound represented by Formula 1 was prepared and isolated as follows.

1. Chaga reduction was made using ratio of 100L reverse osmosis filtered water and 1.5-2 lbs of chopped and ground chaga.

2. This water/chaga mixture is heated to approx. 160°F-190°F and maintained for 3-7 days under slight vacuum in glass reactor vessel.

*The reduction lost up to 5% volume through evaporation/vaporization.

3. Once chaga reduction condensed for 3-7 days the chaga reduction was transferred from the glass reactor to a stainless steel still fitted with a copper helmet.

4. Chaga reduction was then distilled for 3-7 hours and produced 1-5 gallons of distillate. Distillation occurred at approx. 210°F +/- 10°F

5. After distillation the remaining fraction not converted to distillate was used for the reaction. The volume of this remaining fraction was approximately 80% of the starting volume. The temperature of the fraction during the reaction stayed at approximately 190F +/-10F

6. Once reduction was removed from still it was mixed in the following quantities for the final reaction:
4 gallons fraction
250-400g Manuka Honey
115-120 mL MCT oil
5 mL 70/30 H2O/Eth Chaga extraction

All ingredients were mixed together in stainless steel vessel where reaction begins. The initial indication of reaction occurring is the giving off of "sparks" of light, the formation of micelles and giving off of heat and gas. The mixture was then bottled to contain and induce the continued reaction for 4-6 hours while formulation cooled slowly to room temperature. Formulation 1 was stored at ambient temperature for 3-4 weeks without degradation.

### Example 15: Production of the Chemical Compound Represented by Formula 2

The chemical compound represented by Formula 2 was prepared and isolated as follows.

**Chaga reduction to fraction as prepared as in Example 14 was used as the starting material.
1. In a 300 mL Parr reaction vessel at room temperature the following was mixed:
   200 mL Chaga fraction
   15g Proline
   15g Fructose
   5 g Sunflower lecithin (or other FA surfactant)
2. The mixture was stirred gently. Starting Material began to crystalize at room temperature.
3. Reaction vessel was sealed according to manufacturer guidelines.
4. Mixture was heated in fully sealed reaction chamber to 405°F +/- 15°F at 35 PSI +/- 10 PSI.
   *Steps 2-4 took approximately 30-50 minutes
5. Once temperature/pressure was achieved heat source was removed and vessel allowed to cool to a max of 90°F before degassing. **WEAR PPE FOR ALL STEPS IN REACTION**
6. Once at correct temperature pressure release valve was opened and chamber slowly de-gassed.
7. Vessel was opened and liquid separated from solid crystal phases. Liquid phase contains concentration of the fully synthesized compound represented by Formula 2.

As noted above, the present invention is applicable to medicinal, and nutraceutical formulations, chemical compounds and pharmaceutical composition containing the chemical compounds and methods of making and using thereof. Accordingly, the present invention should not be considered limited to the examples described above, but rather should be understood to cover all aspects of the invention as fairly set out in the attached claims.
The present invention is also described in the following clauses.
1. A compound represented by Formula 1 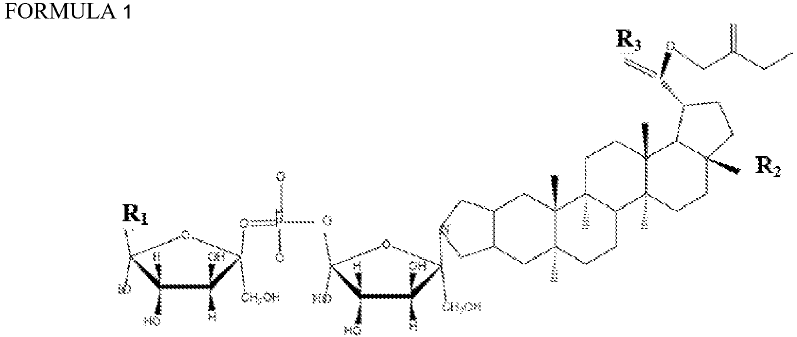 wherein R₁, R₂ and R₃ are, independently, selected from the group consisting of alkyl/alkane, alkene/alkenyl, or alkyne/alkynyl, having from 1 to 20 carbon atom; benzene/aromatic/phenyl, ether, amide, alkyl halide, amine (-amino), alcohol/hydroxy/hydroxyl (-OH), thiol, aldehyde, ketone, ester, carboxylic acid (COOH), acid anhydride, acyl halide, or methyl.
2. The compound of clause 1, wherein at least one of R₁, R₂ and R₃ is a hydroxyl group.
3. The compound of clause 1, wherein R₁, R₂ and R₃ are hydroxyl groups.
4. A compound represented by Formula 2 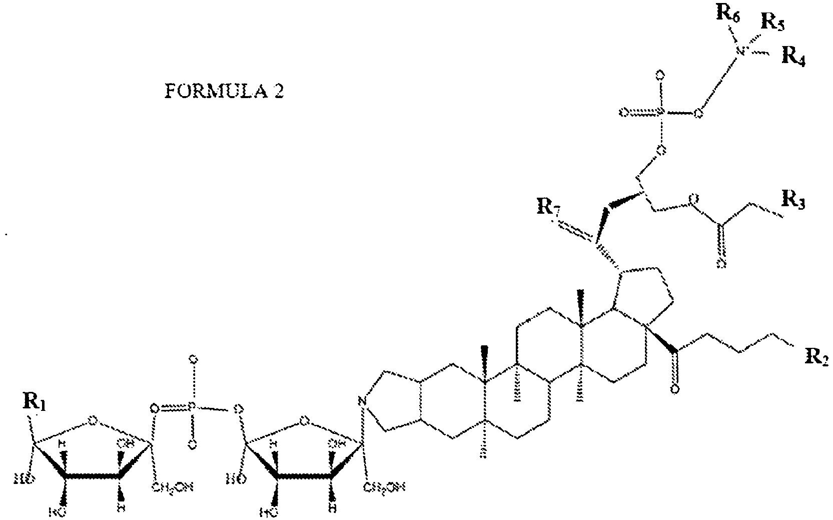 wherein R₁, R₂, R₃, R₄, R₅, R₆, and R₇ are independently selected from the group consisting of alkyl/alkane, alkene/alkenyl, or alkyne/alkynyl, having from 1 to 20 carbon atom; benzene/aromatic/phenyl, ether, amide, alkyl halide, amine (-amino), alcohol/hydroxy/hydroxyl (-OH), thiol, aldehyde, ketone, ester, carboxylic acid (COOH), acid anhydride, acyl halide, or methyl.
5. The compound of clause 4, wherein at least one of R₁ - R₇ is a hydroxyl group.
6. The compound of clause 4, wherein R₁ - R₇ are hydroxyl groups.
7. A pharmaceutical composition comprising the compounds of any one of clauses 1-6 in a pharmaceutically acceptable carrier.
8. The pharmaceutical composition of clause 7 wherein the pharmaceutically acceptable carrier is selected from the group consisting of artificial and biological delivery systems, nano drug delivery carriers, microsystems, vesicles of biological or inert origin, and biological macromolecules.
9. A method of treating a neoplastic disease in a mammalian subject, comprising administering to the subject an effective amount of the compound of any one of clauses 1-6 or the pharmaceutical composition of any one of clauses 7-8.
10. The method of clause 9, wherein the neoplastic disease is cancer.
11. The method of clause 10, wherein the cancer is melanoma, carcinoma, sarcoids, prostate cancer, colorectal cancer, breast cancer, liver cancer and brain cancer.
12. The method of clause 11, wherein the carcinoma is squamous cell carcinoma, basal cell carcinoma, or Merkel-cell carcinoma.
13. A nutraceutical formulation comprising a mixture of a reduction of Chaga mushroom, and an extraction of Chaga mushroom.
14. A method of making a nutraceutical formulation comprising:
   forming a reduction of Chaga mushroom in a reducing solvent;
   forming an extraction of Chaga mushroom in an extraction solvent; and
   mixing the reduction of Chaga mushroom with the extraction of Chaga mushroom to make the nutraceutical formulation.
15. A medicinal formulation comprising a reaction product wherein the reaction product is a product formed by reacting a mixture of a Chaga mushroom reduction and a Chaga mushroom extraction with an esterification mixture comprising proline, fructose and a fatty acid, and an emulsifying sugar ester capable of emulsion within otherwise non-miscible liquids to facilitate emulsion at a temperature that results in a reaction between the Chaga mushroom reduction and Chaga mushroom extraction mixture with the esterification mixture to produce a reaction mixture, and combining the reaction mixture with a medium chain triglyceride oil to make the medicinal formulation.
16. The method of clause 15, wherein the esterification mixture is a Manuka honey.
17. A method of making a medicinal formulation comprising:
   preparing a Chaga mushroom reduction in a reduction solvent under pressure;
   preparing a Chaga mushroom extraction in an extraction solvent;
   mixing the Chaga reduction with the Chaga extraction;
   reacting the Chaga reduction and Chaga extraction mixture with an esterification mixture comprising proline, fructose and a fatty acid, and an emulsifying sugar ester capable of emulsion within otherwise non-miscible liquids to facilitate emulsion at a temperature that results in a reaction between the Chaga mushroom reduction and Chaga mushroom extraction mixture with the esterification mixture to produce a reaction mixture and combining the reaction mixture with medium chain triglyceride oil to make the medicinal formulation.
18. The method of clause 17, wherein the esterification mixture is a Manuka honey.
19. A method of treating a neoplastic disease in a mammalian subject, comprising administering to the subject an effective amount of the medicinal formulation of clause 15.
20. The method of clause 19, wherein the neoplastic disease is cancer.

## Claims

1. A nutraceutical formulation comprising a mixture of a reduction of Chaga mushroom, and an extraction of Chaga mushroom.

2. A method of making a nutraceutical formulation comprising:
forming a reduction of Chaga mushroom in a reducing solvent;
forming an extraction of Chaga mushroom in an extraction solvent; and
mixing the reduction of Chaga mushroom with the extraction of Chaga mushroom to make the nutraceutical formulation.

3. A medicinal formulation comprising a reaction product wherein the reaction product is a product formed by reacting a mixture of a Chaga mushroom reduction and a Chaga mushroom extraction with an esterification mixture comprising proline, fructose and a fatty acid, and an emulsifying sugar ester capable of emulsion within otherwise non-miscible liquids to facilitate emulsion at a temperature that results in a reaction between the Chaga mushroom reduction and Chaga mushroom extraction mixture with the esterification mixture to produce a reaction mixture, and combining the reaction mixture with a medium chain triglyceride oil to make the medicinal formulation.

4. The medicinal formulation of claim 3, wherein the esterification mixture is a Manuka honey.

5. A method of making a medicinal formulation comprising:
preparing a Chaga mushroom reduction in a reduction solvent under pressure;
preparing a Chaga mushroom extraction in an extraction solvent;
mixing the Chaga reduction with the Chaga extraction;
reacting the Chaga reduction and Chaga extraction mixture with an esterification mixture comprising proline, fructose and a fatty acid, and an emulsifying sugar ester capable of emulsion within otherwise non-miscible liquids to facilitate emulsion at a temperature that results in a reaction between the Chaga mushroom reduction and Chaga mushroom extraction mixture with the esterification mixture to produce a reaction mixture and combining the reaction mixture with medium chain triglyceride oil to make the medicinal formulation.

6. The method of claim 5, wherein the esterification mixture is a Manuka honey.

7. The medicinal formulation of claim 3 for use treating a neoplastic disease in a mammalian subject, comprising administering to the subject an effective amount of the medicinal formulation to the subject.

8. The method of claim 7, wherein the neoplastic disease is cancer.
